(19) **Europäisches Patentamt / European Patent Office / Office européen des brevets**

(11) **EP 2 898 091 B1**

(12) **EUROPEAN PATENT SPECIFICATION**

(45) Date of publication and mention of the grant of the patent:
**02.10.2019 Bulletin 2019/40**

(21) Application number: **13766076.7**

(22) Date of filing: **23.09.2013**

(51) Int Cl.:
*C12Q 1/6886* (2018.01)   *A61K 31/7068* (2006.01)

(86) International application number:
**PCT/EP2013/069736**

(87) International publication number:
**WO 2014/044848 (27.03.2014 Gazette 2014/13)**

(54) **METHODS FOR PREDICTING MULTIPLE MYELOMA TREATMENT RESPONSE**

VERFAHREN ZUR VORHERSAGE DER BEHANDLUNGSREAKTION VON MULTIPLEM MYELOM

PROCÉDÉS POUR PRÉDIRE UNE RÉPONSE DE TRAITEMENT D'UN MYÉLOME MULTIPLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.09.2012 EP 12306141**

(43) Date of publication of application:
**29.07.2015 Bulletin 2015/31**

(73) Proprietors:
- **Institut National de la Santé et de la Recherche Médicale (INSERM)**
  **75013 Paris (FR)**
- **Université de Montpellier I**
  **34967 Montpellier Cedex 2 (FR)**
- **Centre Hospitalier Universitaire de Montpellier**
  **34295 Montpellier Cedex 05 (FR)**

(72) Inventors:
- **MOREAUX, Jérôme**
  **F-34295 Montpellier Cedex 5 (FR)**
- **KLEIN, Bernard**
  **F-34295 Montpellier Cedex 5 (FR)**

(74) Representative: **Monni, Richard**
  **Cabinet LLR**
  **11 boulevard de Sébastopol**
  **75001 Paris (FR)**

(56) References cited:
- **J. MOREAUX ET AL: "A high-risk signature for patients with multiple myeloma established from the molecular classification of human myeloma cell lines", HAEMATOLOGICA, vol. 96, no. 4, 20 December 2010 (2010-12-20), pages 574-582, XP055050703, ISSN: 0390-6078, DOI: 10.3324/haematol.2010.033456 cited in the application -& J. Moreaux ET AL: "A high-risk signature for patients with multiple myeloma established from the molecular classification of human myeloma cell lines- supplementary appendix", Haematologica, 20 December 2010 (2010-12-20), pages 1-14, XP055051624, DOI: 10.3324/haematol.2010.033456 Retrieved from the Internet: URL:http://www.haematologica.org/content/s uppl/2011/04/01/haematol.2010.033456.DC2/0 33456.Moreaux_suppl.pdf [retrieved on 2013-01-29]**
- **K. Vanderkerken ET AL: "EPIGENETIC CHANGES OF MYELOMA CELLS WITHIN THE BONE MARROW MICROENVIRONMENT", Haematologica: Abstract Book 13th International Myeloma Workshop, Paris, France, May 3-6, 2011, vol. 96, no. supplement 1 1 May 2011 (2011-05-01), pages s8-s9, XP055051689, ISSN: 0390-6078 Retrieved from the Internet: URL:http://www.haematologica.org/content/9 6/supplement_1/S1.full.pdf+html [retrieved on 2013-01-30]**

**(Cont. next page)**

- G. HELLER ET AL: "Genome-Wide Transcriptional Response to 5-Aza-2'-Deoxycytidine and Trichostatin A in Multiple Myeloma Cells", CANCER RESEARCH, vol. 68, no. 1, 1 January 2008 (2008-01-01), pages 44-54, XP055051386, ISSN: 0008-5472, DOI: 10.1158/0008-5472.CAN-07-2531
- EMMA M. SMITH ET AL: "The potential role of epigenetic therapy in multiple myeloma", BRITISH JOURNAL OF HAEMATOLOGY, vol. 148, no. 5, 1 March 2010 (2010-03-01) , pages 702-713, XP055051410, ISSN: 0007-1048, DOI: 10.1111/j.1365-2141.2009.07976.x
- M. NOJIMA ET AL: "Genomic Screening for Genes Silenced by DNA Methylation Revealed an Association between RASD1 Inactivation and Dexamethasone Resistance in Multiple Myeloma", CLINICAL CANCER RESEARCH, vol. 15, no. 13, 1 July 2009 (2009-07-01), pages 4356-4364, XP055050720, ISSN: 1078-0432, DOI: 10.1158/1078-0432.CCR-08-3336

**Description**

**FIELD OF THE INVENTION:**

The present invention relates to methods for predicting multiple myeloma treatment response.

**BACKGROUND OF THE INVENTION:**

Malignant transformation requires oncogenic activation and inactivation of tumor suppressor genes, which help cancer cells overriding the normal mechanisms controlling cellular survival and proliferation (1,2). These molecular events are caused by genetic alterations (translocations, amplification, mutations) and also by epigenetic modifications (3). Epigenetic modifications include methylation of DNA cytosine residues and histone modifications and have been shown to be critical in the initiation and progression of cancers (4). DNA methyltransferase inhibitors or HDAC inhibitors are now being used in the treatment of several hematologic malignancies including MM (5-8).

Multiple myeloma (MM) is a plasma cell neoplasm characterized by the accumulation of malignant plasma cells, termed Multiple Myeloma Cells (MMCs) within the bone marrow (BM). Despite the recent introduction of therapies such as Lenalidomide and Bortezomib, MM remains an almost incurable disease. MM arises through the accumulation of multiple genetic changes that include an aberrant or overexpression of a D-type cyclin gene, cyclin D1 (CCND1) in the case of t(11;14) translocation or gain in 11q13, cyclin D3 (CCND3) in the case of the rare t(6;14) translocation, or cyclin D2 (CCND2) on the background of a translocation involving *c-maf* (t(14;16)) or MMSET/*FGFR3* (t(4;14)) (9,10).

Recent studies have shown that epigenetic changes such as DNA methylation play a role by silencing various cancer-related genes in MM. Most of these studies have been performed on limited number of genes using methylation specific PCR (11-18). Among the genes identified with promoter hypermethylation in MM, cyclin-dependent kinase inhibitor 2A (*CDKN2A*) and transforming growth factor beta receptor 2 (*TGFBR2*) have been shown to be associated with a poor prognosis in MM patients with discrepant results for *CDKN2A* (*12*). Heller at al. have identified several epigenetic inactivated cancer related genes in 3 human myeloma cell lines (HMCLs) and validated the relevance of 10 of these genes in 6 additional HMCLs, premalignant PCs from 24 MGUS patients and MMCs from 111 patients with MM (19). Methylation of secreted protein acidic and rich in cysteine (*SPARC*) and Bcl2/adenovirus E1B 19kDa interacting protein 3 (B*NIP3*) promoters was associated with poor overall survival of MM patients (19). SOCS3 methylation was found to be associated with extramedullary manifestations, plasma cell leukemia and significant shortened survival in MM patients (20). More recently, Morgan *et al* have shown that the transition of normal plasma cell (PC) and MGUS stage to MM stage is associated with DNA hypomethylation, but the transition of intramedullary MM stage to plasma cell leukemia or HMCL stage is associated with DNA hypermethylation (21). They described 2 specific subgroups of hyperdiploid MM on the basis of their methylation profile, which had a significantly different overall survival (21).

DNMT inhibitors can be sub-divided into nucleoside analogue and non-nucleoside analogue families. 5-Azacytidine(azacytidine)/5-Aza-2'-deoxycytidine (decitabine) are both nucleoside analogues with Food and Drug Administration approval for use in myelodysplastic syndrome. Clinical trials in myeloma combining these demethylating agents with chemotherapy or other agents are underway (8,72).

An important objective for optimizing these clinical trials will be the identification of biomarkers predictive for sensitivity of MMCs to DNMTi. Expression marker signatures to classify and characterize Multiple Myeloma have already been described (22,71). In the present invention, the inventors used gene expression profiling of Multiple Myeloma Cells (MMCs) to build a novel "DNA methylation gene expression score" that makes it possible identification of patients whose MMCs will be targeted by DNMT inhibition.

**SUMMARY OF THE INVENTION:**

The present invention relates to a method of testing whether a patient suffering of multiple myeloma will respond or not to a DNA methyltransferase inhibitor (DNMTi).

**DETAILED DESCRIPTION OF THE INVENTION:**

The multiple myeloma treatment response was investigated by the inventors using DNA methyltransferase inhibitors (DNMTi), human multiple myeloma cell lines and primary myeloma cells. The inventors analyzed gene expression profiles of 5 MM cell lines treated with decitabine using microarrays. The inventors identified 127 genes deregulated by decitabine. 47 out of 127 decitabine deregulated genes have prognostic value in inventor's cohort of 206 newly-diagnosed MM patients. The inventors summarized the prognostic information in a DNA methylation score (DM Score or DMS) built using the probe set signal value weighted by the beta coefficient of prognostic genes. The DM Score is predictive for DNMT inhibitor sensitivity of HMCLs and primary myeloma cells *in vitro.* The DM Score allows identification of myeloma patients that could benefit DNMT inhibitor treatment.

The inventors also demonstrated that the DM Score is also predictive of myeloma cells sensitivity for another DNMTi, 5-Azacytidine. HMCLs with a high DM Score exhibited significant 3-fold higher 5-Azacytidine sensitivity than HMCLs with a low DM Score and these results were validated in vitro with primary myeloma cells of patients.

## *Definitions*

The term "patient" denotes a mammal. In a preferred embodiment of the invention, a patient refers to any patient (preferably human) afflicted with multiple myeloma. The term "multiple myeloma" refers to multiple myeloma such as revised in the World Health Organisation Classification C90.

The term "DNA methyltransferase inhibitors" or "DNMTi" has its general meaning in the art and refers to a multiple myeloma treatment. The term "DNA methyltransferase inhibitors" or "DNMTi" refers to DNA methyltransferase inhibitor that can be sub-divided into nucleoside analogue (5-Azacytidine (azacytidine), 5-Aza-2'-deoxycytidine (decitabine, 5-Aza-CdR), zebularine, 5-Fluoro-2'-deoxycytidine (5-F-CdR), 5 ,6-Dihydro-5-azacytidine (DHAC)) and non-nucleoside analogue families (Hydralazine, Procainamide, Procaine, EGCG ((-)-epigallocatechin-3-gallate), Psammaplin A, MG98, RG108) (8).

The term "biological sample" refers to multiple myeloma cells, bone marrow or medullary cell.

All the genes pertaining to the invention are known per se, and are listed in the below Table A.

Table A: Set of predictive genes.

| Gene | Gene Symbol | Gene name | Gene ID | β coefficient | Reference level (ELRi) |
|---|---|---|---|---|---|
| G1 | COP1 | Caspase-1 dominant-negative inhibitor pseudo-ICE | 1552701_a_ at | 0,669962539 | 25.72815534 |
| G2 | DNAJB9 | DnaJ (Hsp40) homolog; subfamily B; member 9 | 1554462_a_ at | -0,807267505 | 87.37864078 |
| G3 | INSIG1 | insulin induced gene 1 | 201625_s_at | 0,918185816 | 10.19417476 |
| G4 | SEL1L | sel-1 suppressor of lin-12-like (C. elegans) | 202061_s_at | -0,683515307 | 86.40776699 |
| G5 | FNDC3A | fibronectin type III domain containing 3A | 202304_at | -0,821182841 | 76.21359223 |
| G6 | IFI27 | interferon, alpha-inducible protein 27 | 202411_at | 0,87241554 | 60.19417476 |
| G7 | STCH | stress 70 protein chaperone; microsome-associated; 60kDa | 202558_s_at | -0,924845543 | 27.66990291 |
| G8 | GALNT3 | UDP-N-acetyl-alpha-D-galactosamine: polypeptide N-acetylgalactosaminyltransf erase 3 (GalNAc-T3) | 203397_s_at | 0,838104028 | 30.58252427 |
| G9 | TPST2 | tyrosylprotein sulfotransferase 2 | 204079_at | 0,698525183 | 14.5631068 |
| G10 | KIF21B | kinesin family member 21B | 204411_at | 1,057242019 | 26.21359223 |
| G11 | G1P2 | Interferon; alpha-inducible protein (clone IFI-15K) | 205483_s_at | 0,704117799 | 40.77669903 |
| G12 | OAS1 | 2',5'-oligoadenylate synthetase 1 | 205552_s_at | 0,918506668 | 12.13592233 |
| G13 | ITGB7 | integrin, beta 7 | 205718_at | 1,393658338 | 11.16504854 |
| G14 | SP110 | SP110 nuclear body protein | 208012_x_at | 0,796781189 | 13.10679612 |
| G15 | EIF2S2 | eukaryotic translation initiation factor 2, subunit 2 beta | 208725_at | -0,608613084 | 75.24271845 |
| G16 | CORO1A | coronin, actin binding protein, 1A | 209083_at | 1,116949242 | 50.48543689 |
| G17 | TUBA3 | tubulin, alpha 1a | 209118_s_at | 0,794556227 | 15.04854369 |
| G18 | ADFP | Adipose differentiation-related protein | 209122_at | -0,742527707 | 73.30097087 |

(continued)

| Gene | Gene Symbol | Gene name | Gene ID | β coefficient | Reference level (ELRi) |
|---|---|---|---|---|---|
| G19 | IFI35 | interferon-induced protein 35 | 209417_s_at | 0,608446212 | 29.12621359 |
| G20 | STAT1 | signal transducer and activator of transcription 1 | 209969_s_at | 0,865200608 | 20.87378641 |
| G21 | HIST1H2BG | histone cluster 1, H2bc | 210387_at | -0,713207906 | 78.15533981 |
| G22 | HLA-DPA1 | major histocompatibility complex, class II, DP alpha 1 | 211990_at | -0,708484927 | 79.12621359 |
| G23 | RECQL | RecQ protein-like (DNA helicase Q1-like) | 213878_at | 0,79702138 | 19.90291262 |
| G24 | CCPG1 | cell cycle progression 1 | 214152_at | -0,686867998 | 48.05825243 |
| G25 | NFE2L1 | nuclear factor (erythroid-derived 2)-like 1 | 214179_s_at | 0,694584139 | 12.13592233 |
| G26 | PARP12 | poly (ADP-ribose) polymerase family, member 12 | 218543_s_at | 1,257650329 | 10.19417476 |
| G27 | FKBP 11 | FK506 binding protein 11; 19 kDa | 219118_at | -0,88381802 | 76.69902913 |
| G28 | EAF2 | ELL associated factor 2 | 219551_at | -0,762323817 | 83.98058252 |
| G29 | C6orf48 | chromosome 6 open reading frame 48 | 220755_s_at | -0,832289767 | 23.78640777 |
| G30 | IL21R | interleukin 21 receptor | 221658_s_at | 0,612993436 | 24.75728155 |
| G31 | PGM3 | Phosphoglucomutase 3 | 221788_at | -1,227167702 | 87.86407767 |
| G32 | EIF2C2 | Eukaryotic translation initiation factor 2C; 2 | 222294_s_at | -0,657954783 | 69.90291262 |
| G33 | TMEM39A | transmembrane protein 39A | 222690_s_at | 0,787711031 | 64.5631068 |
| G34 | SLAMF7 | SLAM family member 7 | 222838_at | -0,601257629 | 64.0776699 |
| G35 | MYLIP | myosin regulatory light chain interacting protein | 223129_x_at | 0,687458269 | 22.33009709 |
| G36 | PPAPDC1B | phosphatidic acid phosphatase type 2 domain containing 1B | 223569_at | -0,860377506 | 83.49514563 |
| G37 | ARHGAP9 | Rho GTPase activating protein 9 /// Rho GTPase activating protein 9 | 224451_x_at | -0,731399299 | 83.98058252 |
| G38 | RPL37 | ribosomal protein L37 | 224763_at | -1,135732424 | 88.34951456 |
| G39 | GSK3B | Glycogen synthase kinase 3 beta | 226183_at | -0,772104529 | 83.98058252 |
| G40 | ELL2 | elongation factor; RNA polymerase II; 2 | 226982_at | -0,687762574 | 80.09708738 |
| G41 | EST, GenBank AA042983 | Expression sequence tag, GenBank AA042983 | 227755_at | -0,79716878 | 68.93203883 |
| G42 | SAMD9 | sterile alpha motif domain containing 9 | 228531_at | 0,813378182 | 46.60194175 |
| G43 | GNG7 | guanine nucleotide binding protein (G protein); gamma 7 | 228831_s_at | -0,641328827 | 80.09708738 |
| G44 | SEC63 | SEC63 Homolog (S. cerevisiae) | 229969_at | -1,216057852 | 85.9223301 |
| G45 | EST, GenBank AI702465 | Expression sequence tag, GenBank AI702465 | 230570_at | -0,659929394 | 82.52427184 |

(continued)

| Gene | Gene Symbol | Gene name | Gene ID | β coefficient | Reference level (ELRi) |
|------|------|------|------|------|------|
| G46 | MGC15875 | hypothetical protein MGC15875 | 232488_at | -1,036621429 | 80.09708738 |
| G47 | SFN | stratifin | 33322_i_at | 0,721955343 | 12.62135922 |

## *Methods for predicting response*

The present invention relates to a method of testing whether a patient suffering of multiple myeloma will respond or not to a DNA methyltransferase inhibitor (DNMTi) comprising:

i) determining the expression level (ELi) of several genes $G_1$-$G_n$ selected from table A in a biological sample obtained from said patient
ii) comparing the expression level (ELi) determined at step i) with a predetermined reference level (ELRi)
iii) calculating the DMS score trough the following formula

$$DMS = \sum_{i=1}^{n} \beta i \times Ci$$

wherein $\beta i$ represent the regression β coefficient reference value for the gene $G_i$ and $Ci$ = 1 if the expression of the gene $G_i$ (ELi) is higher than the predetermined reference level (ELRi) or $Ci$ = -1 if the expression of the gene (ELi) is lower than or equal to the predetermined reference level (ELRi)
iv) comparing the score DMS determined at step iii) with a predetermined reference value $DMS_R$
v) and concluding that the patient will respond to the DNMTi when the DMS score is higher than the predetermined reference value $DMS_R$ or concluding that the patient will not respond to the DNMTi when the DMS score is lower than the predetermined reference value $DMS_R$

In some aspects, the levels of at least 25 genes from Table A are determined wherein said genes are:

202061_s_at SEL1L
202304_at FNDC3A
202558_s_at STCH
203397_s_at GALNT3
204079_at TPST2
205483_s_at G1P2
205552_s_at OAS1
205718_at ITGB7
208725_at EIF2S2
209083_at CORO1A
209118_s_at TUBA3
209122_at ADFP
209417_s_at IFI35
211990_at HLA-DPA1
214152_at CCPG1
214179_s_at NFE2L1
219118_at FKBP11
221658_s_at IL21R
222690_s_at TMEM39A
223129_x_at MYLIP
224451_x_at ARHGAP9
224763_at RPL37
227755_at EST, GenBank AA042983
228831_s_at GNG7

232488_at MGC15875

In some aspects, the level of 25, 26, 27, 28, 29, 30, 31, 32, 33, 34, 35, 36, 37, 38, 39, 40, 41, 42, 43, 44, 45, 46, or 47 genes from Table A are determined wherein every combinations of genes comprises a minimal set of 25 genes consisting of:

202061_s_at SEL1L
202304_at FNDC3A
202558_s_at STCH
203397_s_at GALNT3
204079_at TPST2
205483_s_at G1P2
205552_s_at OAS1
205718_at ITGB7
208725_at EIF2S2
209083_at CORO1A
209118_s_at TUBA3
209122_at ADFP
209417_s_at IFI35
211990_at HLA-DPA1
214152_at CCPG1
214179_s_at NFE2L1
219118_at FKBP11
221658_s_at IL21R
222690_s_at TMEM39A
223129_x _at MYLIP
224451_x_at ARHGAP9
224763_at RPL37
227755_at EST, GenBank AA042983
228831_s_at GNG7
232488_at MGC15875

In some embodiments, the level of the 47 genes of Table A are determined.

Determination of the expression level of the genes can be performed by a variety of techniques. Generally, the expression level as determined is a relative expression level. More preferably, the determination comprises contacting the biological sample with selective reagents such as probes, primers or ligands, and thereby detecting the presence, or measuring the amount, of polypeptide or nucleic acids of interest originally in the biological sample. Contacting may be performed in any suitable device, such as a plate, microtiter dish, test tube, well, glass, column, and so forth. In specific aspects, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or polypeptides of the biological sample.

In a preferred aspect, the expression level may be determined by determining the quantity of mRNA.

Methods for determining the quantity of mRNA are well known in the art. For example the nucleic acid contained in the biological sample is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Other methods of amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain aspects, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin) .

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In a particular aspect, the methods of the invention comprise the steps of providing total RNAs extracted from a biological samples and subjecting the RNAs to amplification and hybridization to specific probes, more particularly by means of a quantitative or semi-quantitative RT-PCR.

In another preferred aspect, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a biological sample from a test patient, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210)

In this context, the invention further provides a DNA chip comprising a solid support which carries nucleic acids that are specific to the genes listed in table A.

Predetermined reference values ELRi or DMS$_R$ used for comparison may consist of "cut-off" values.

For example; each reference ("cut-off') value ELRi for each gene may be determined by carrying out a method comprising the steps of:

a) providing a collection of samples from patients suffering of multiple myeloma;
b) determining the expression level of the relevant gene for each sample contained in the collection provided at step a);
c) ranking the samples according to said expression level
d) classifying said samples in pairs of subsets of increasing, respectively decreasing, number of members ranked according to their expression level,
e) providing, for each sample provided at step a), information relating to the actual clinical outcome for the corresponding cancer patient (i.e. the duration of the disease-free survival (DFS) or the overall survival (OS) or both);
f) for each pair of subsets of tumour tissue samples, obtaining a Kaplan Meier percentage of survival curve;
g) for each pair of subsets of tumour tissue samples calculating the statistical significance (p value) between both subsets
h) selecting as reference value ELR for the expression level, the value of expression level for which the p value is the smallest.

For example the expression level of a gene Gi has been assessed for 100 samples of 100 patients. The 100 samples are ranked according to the expression level of gene Gi. Sample 1 has the highest expression level and sample 100 has the lowest expression level. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding cancer patient, Kaplan Meier curves are prepared for each of the 99 groups of two subsets. Also for each of the 99 groups, the p value between both subsets was calculated. The reference value ELRi is then selected such as the discrimination based on the criterion of the minimum p value is the strongest. In other terms, the expression level corresponding to the boundary between both subsets for which the p value is minimum is considered as the reference value. It should be noted that according to the experiments made by the inventors, the reference value ELRi is not necessarily the median value of expression levels.

The man skilled in the art also understands that the same technique of assessment of the DMS$_R$ could be used for obtaining the reference value and thereafter for assessment of the response to DNMTi. However in one embodiment, the reference value $DMS_R$ is the median value of DMS.

In one aspect, the reference value ELRi for the genes are described in table A (right column).

In one aspect, the reference value $DMS_R$ is -19.7 for determining whether a patient suffering of multiple myeloma will respond to a DNMTi or -15.3 for predicting the survival time of patient suffering of multiple myeloma.

The regression β coefficient reference values may be easily determined by the skilled man in the art for each gene using a Cox model. The Cox model is based on a modeling approach to the analysis of survival data. The purpose of the model is to simultaneously explore the effects of several variables on survival. The Cox model is a well-recognised statistical technique for analysing survival data. When it is used to analyse the survival of patients in a clinical trial, the model allows us to isolate the effects of treatment from the effects of other variables. The logrank test cannot be used to explore (and adjust for) the effects of several variables, such as age and disease duration, known to affect survival. Adjustment for variables that are known to affect survival may improve the precision with which we can estimate the treatment effect. The regression method introduced by Cox is used to investigate several variables at a time. It is also known as proportional hazards regression analysis. Briefly, the procedure models or regresses the survival times (or more specifically, the so-called hazard function) on the explanatory variables. The hazard function is the probability that an individual will experience an event (for example, death) within a small time interval, given that the individual has survived up to the beginning of the interval. It can therefore be interpreted as the risk of dying at time **t**. The quantity h0 (**t**) is the baseline or underlying hazard function and corresponds to the probability of dying (or reaching an event) when all the explanatory variables are zero. The baseline hazard function is analogous to the intercept in ordinary regression (since exp0= 1). The regression coefficient β gives the proportional change that can be expected in the hazard, related to changes in the explanatory variables. The coefficient β is estimated by a statistical method called maximum likelihood. In survival analysis, the hazard ratio (HR) (Hazard Ratio = exp(β)) is the ratio of the hazard rates corresponding to the conditions described by two sets of explanatory variables. For example, in a drug study, the treated population may die at twice the rate per unit time as the control population. The hazard ratio would be 2, indicating higher hazard of death from the treatment.

In one aspect, the regression β coefficient reference values are described in table A.

Also disclosed is a kit for performing the methods as above described, wherein said kit comprises means for measuring the expression level of the genes listed in Table A. Typically the kit may include a primer, a set of primers, a probe, a set of probes as above described. In a particular embodiment, the probe or set of probes are labelled as above described. The kit may also contain other suitably packaged reagents and materials needed for the particular detection protocol, including solid-phase matrices, if applicable, and standards.

In a particular aspect, the score may be generated by a computer program.

### *Methods of treatment*

The method of the invention allows to define a subgroup of patients who will be responsive ("responder") or not ("non responder") to the treatment with a DNA methyltransferase inhibitor.

Also disclosed is a method for the treatment of multiple myeloma in a patient in need thereof.

In the context of the disclosure, the term "treating" or "treatment", as used herein, means reversing, alleviating, inhibiting the progress of, or preventing the disorder or condition to which such term applies, or one or more symptoms of such disorder or condition.

In a particular aspect, the method comprises the following steps

a) testing whether the patient will respond or not to a DNA methyltransferase inhibitor (DNMTi) by performing the method according to the invention

b) administering the DNA methyltransferase inhibitor, if said patient has as score higher than the reference value DMSR (i.e. the patient will respond to the DNA methyltransferase inhibitor).

A further object of the invention relates to a DNA methyltransferase inhibitor for use in the treatment of multiple myeloma in a patient in need thereof, wherein the patient was being classified as responder by the method as above described.

The invention will be further illustrated by the following figures and examples. However, these examples and figures should not be interpreted in any way as limiting the scope of the present invention.

**FIGURES:**

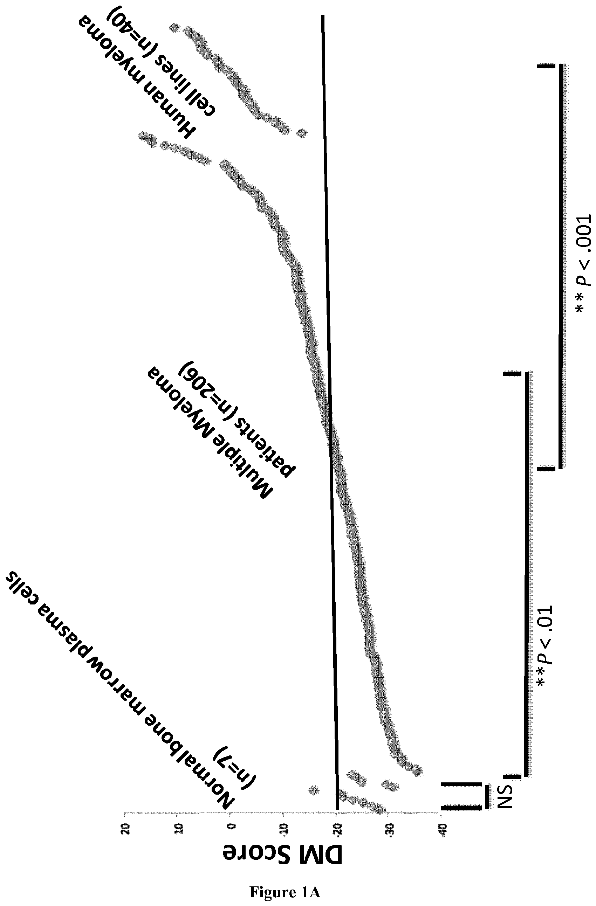

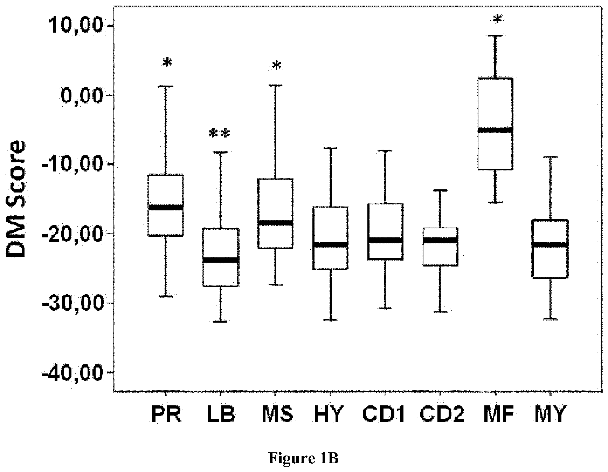

**Figure 1: DNA methylation Score in normal and malignant plasma cells**

(A) DNA methylation Score in normal bone marrow plasma cells (N = 7), in premalignant plasma cells of patients with monoclonal gammopathy of undetermined significance (MGUS, N = 5), in multiple myeloma cells of patients with intramedullary MM (N = 206) and in human myeloma cell lines (N = 40). ** Indicate that the score value is significantly different with a *P* value < .01. (B) DNA methylation Score in the 8 groups of the molecular classification of multiple myeloma. The DM Score was investigated in the 8 groups of the molecular classification of multiple myeloma in UAMS-TT2 cohort of patients. PR: proliferation, LB: low bone disease, MS: MMSET, HY: hyperdiploid, CD1: Cyclin D1, CD2: Cyclin D2, MF: MAF, MY: myeloid. * Indicate that the score value is significantly higher in the group compared to all the patients of the cohort (*P* < .05). ** Indicate that the score value is significantly lower in the group compared to all the patients of the cohort (*P* < .05).

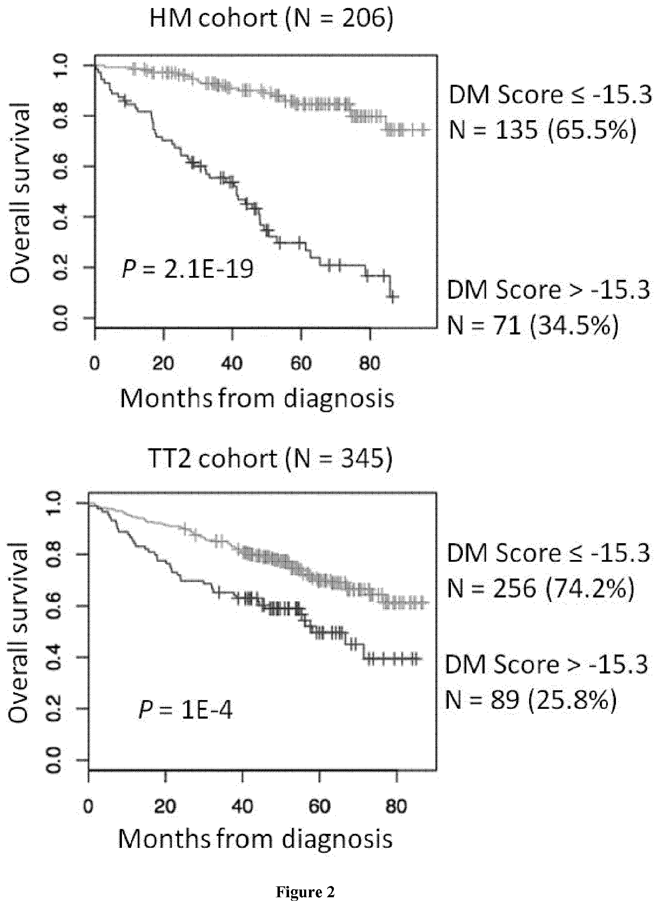

**Figure 2: Prognostic value of DM Score in multiple myeloma.**
Patients of HM cohort were ranked according to increased DM Score and a maximum difference in OS was obtained with DM Score = -15.3 splitting patients in a high risk (34.5%) and low risk (65.5%) groups. The prognostic value of DM Score was tested on an independent cohort of 345 patients from UAMS treated with TT2 therapy (UAMS- TT2 cohort). The parameters to compute the DM Score of patients of UAMS-TT2 cohort and the DM Score cut-off delineating the 2 prognostic groups were those defined with HM cohort.

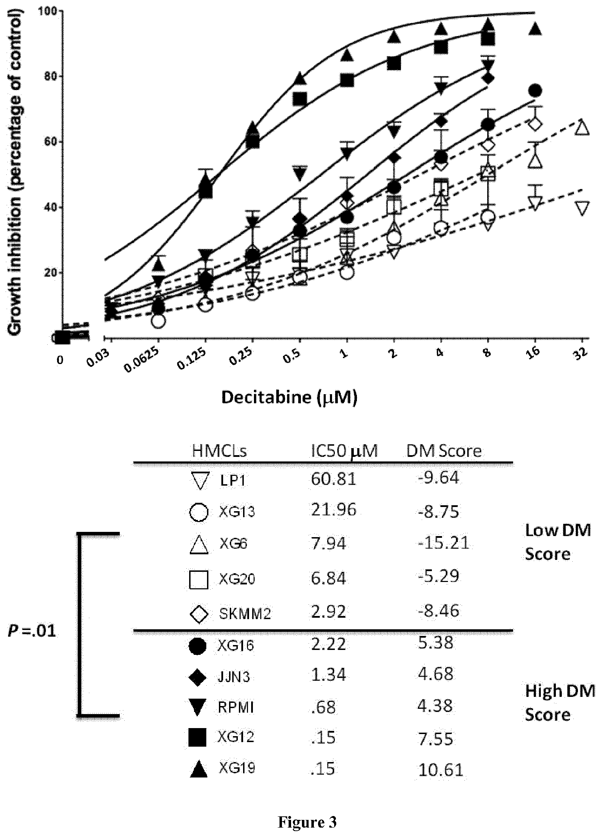

**Figure 3: DM Score predicts for sensitivity of human myeloma cell lines to decitabine.**
(A) HMCLs with high DM Score (N = 5) exhibit significant higher DNMTi sensitivity compared to HMCLs with low DM Score (N = 5). HMCLs were cultured for 4 days in 96-well flat-bottom microtiter plates in RPMI 1640 medium, 10% FCS, 2 ng/ml IL-6 culture medium (control), with graded decitabine concentrations. Data are mean values plus or minus standard deviation (SD) of 5 experiments determined on sextuplet culture wells.

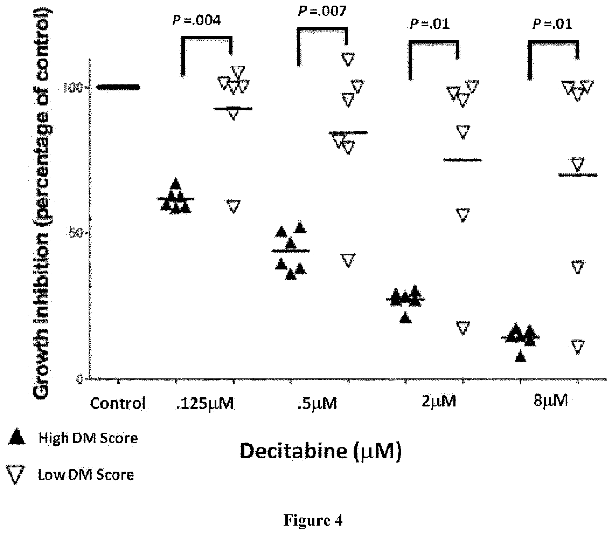

**Figure 4: DM Score predicts for decitabine sensitivity of primary myeloma cells of patients.**
Mononuclear cells from tumor samples of 12 patients with MM were cultured for 4 days in the presence of IL-6 (2 ng/ml) with or without graded decitabine concentrations. At day 4 of culture, the cell count and the viability were determined and the percentage of CD138[+] viable plasma cells was determined by flow cytometry. Black color represents patients with high DM Score (N = 6) and white represents patients with low DM Score values (N = 6).

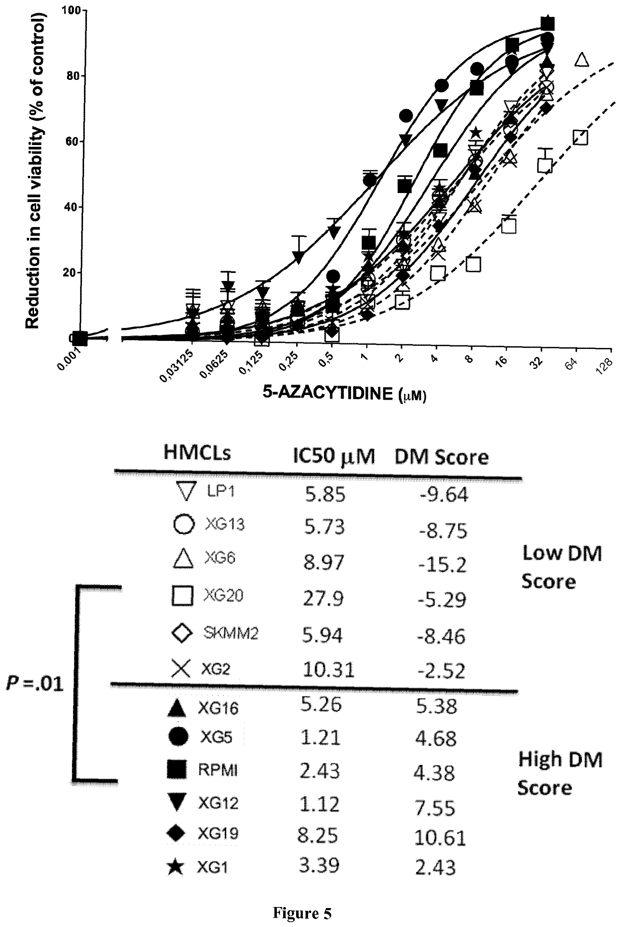

**Figure 5: DM Score predicts for sensitivity of human myeloma cell lines to 5-azacitidine.**
HMCLs with a high DM Score (n = 6) exhibit significant higher 5-azacitidine sensitivity compared to HMCLs with a low DM Score (n = 6). HMCLs were cultured for 4 days in 96-well flat-bottom microtiter plates in RPMI 1640 medium, 10% FCS, 2 ng/ml IL-6 culture medium (control), and graded 5-azacitidine concentrations. Data are mean values plus or minus standard deviation (SD) of 5 experiments determined on sextuplet culture wells.

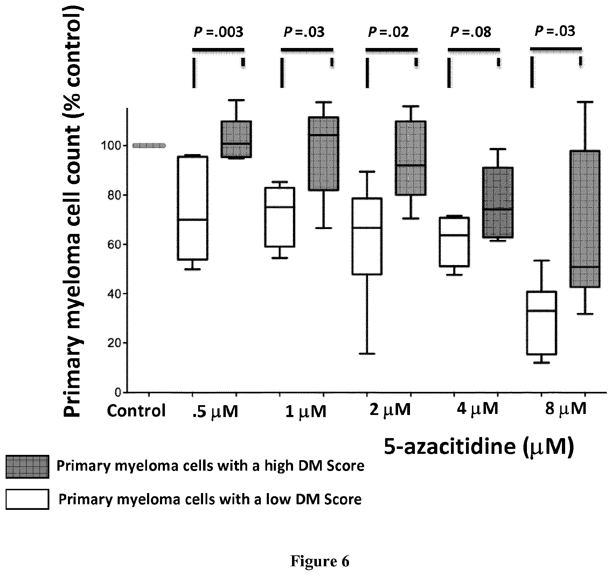

**Figure 6: DM Score predicts for sensitivity of primary myeloma cells of patients to 5-azacitidine.**
Mononuclear cells from tumor samples of 14 patients with MM were cultured for 4 days in the presence of IL-6 (2 ng/ml) with or without graded 5-azacitidine concentrations. At day 4 of culture, the count of viable CD138[+] MMCs was determined using flow cytometry. The black columns represent the mean $\pm$ SD of primary myeloma cell counts (expressed as the percentage of the count without adding 5-azcytidine) of the 7 patients with a low DM Score and the white columns that of the 7 patients with a high DM Score.

## EXAMPLE 1: DEVELOPMENT OF GENE EXPRESSION BASED SCORE TO PREDICT SENSITIVITY OF MULTIPLE MYELOMA CELLS TO DNA METHYLATION INHIBITORS

### Material & Methods

### Human Myeloma Cell Lines (HMCLs)

XG-1, XG-2, XG-3, XG-4, XG-5, XG-6, XG-7, XG-10, XG-11, XG-12, XG-13, XG-14, XG-16, XG-19, XG-20 and XG-21 human myeloma cell lines were obtained as previously described (22-26). JJN3 was kindly provided by Dr Van Riet (Bruxelles, Belgium), JIM3 by Dr MacLennan (Birmingham, UK) and MM1S by Dr S. Rosen (Chicago, USA). AMO-1, LP1, L363, U266, OPM2, and SKMM2 were from DSMZ (Germany) and RPMI8226 from ATTC (USA). All HMCLs derived in inventor's laboratory were cultured in the presence of recombinant IL-6. HMCLs microarray data have been deposited in the ArrayExpress public database under accession numbers E-TABM-937 and E-TABM-1088.

**Primary multiple myeloma cells**

MMCs were purified from 206 patients with newly-diagnosed MM after written informed consent was given at the University hospitals of Heidelberg (Germany) or Montpellier (France) and agreement of the Center for Biological Resources of Montpellier University Hospital (N°DC-2008-417). The study was approved by the ethics boards of Heidelberg and Montpellier Universities. These 206 patients were treated with high dose Melphalan (HDM) and autologous stem cell transplantation (ASCT) (27) and were termed in the following Heidelberg-Montpellier (HM) series (Supplementary Table S1). The CEL files and MAS5 files have been deposited in the ArrayExpress public database (E-MTAB-372). The structural chromosomal aberrations including t(4;14)(p16.3;q32.3) and t(11;14)(q13;q32.3), as well as numerical aberrations including 17p13 and 1q21 gain, were assayed by fluorescence in situ hybridization (iFISH) (28). The inventors also used Affymetrix data of a cohort of 345 purified MMC from previously untreated patients from the University of Arkansas for Medical Sciences (UAMS, Little Rock, AR). The patients were treated with total therapy 2 including HDM and ASCT (29) and termed in the following UAMS-TT2 series. These data are publicly available via the online Gene Expression Omnibus (Gene Expression Profile of Multiple Myeloma, accession number GSE2658. http://www.ncbi.nlm.nih.gov/geo/). As iFISH data were not available for UAMS-TT2 patients, t(4;14) translocation was evaluated using *MMSET* spike expression (30) and del17p13 surrogated by *TP53* probe set signal (31). After Ficoll-density gradient centrifugation, plasma cells were purified using anti-CD138 MACS microbeads (Miltenyi Biotech, Bergisch Gladbach, Germany).

**Cell culture and treatment for gene expression profiling**

The human MM cell lines XG-5, XG-6, XG-7, XG-20 and LP1 were grown in RPMI 1640 supplemented with 10% fetal bovine serum and 2 ng/ml recombinant IL-6. Cells ($2 \times 10^5$/mL) were treated either with 0.5 $\mu$mol/L 5-Aza-2'-deoxycytidine (decitabine) (Sigma, St Louis, MO) for 7 days. Control cells were cultured in the same conditions without adding drug.

**Growth assay for myeloma cells**

HMCLs were cultured for 4 days in 96-well flat-bottom microtiter plates in RPMI 1640 medium, 10% FCS, 2 ng/ml IL-6 culture medium (control), with graded decitabine concentrations. Cell growth was evaluated by quantifying intracellular ATP amount with a Cell Titer Glo Luminescent Assay (Promega, Madison, WI) with a Centro LB 960 luminometer (Berthold Technologies, Bad Wildbad, Germany).

**Mononuclear cell culture**

Mononuclear cells from tumor samples of 12 patients with MM (agreement of the Center for Biological Resources of Montpellier University Hospital (N°DC-2008-417)) were cultured for 4 days at $2 \times 10^5$ cells/ml in RPMI 1640 medium, 10% FCS, 2 ng/ml IL-6, with or without graded concentrations of decitabine. In each culture group, viability and cell counts were assayed and MMCs were stained with an anti-CD 138-PE mAb (Immunotech, Marseille, France) as previously described (32).

**Preparation of complementary RNA (cRNA) and microarray hybridization**

RNA was extracted using the RNeasy Kit (Qiagen, Hilden, Germany) as previously described (33,34). Biotinylated cRNA was amplified with a double *in vitro* transcription and hybridized to the human U133 2.0 plus GeneChips, according to the manufacturer's instructions (Affymetrix, Santa Clara, CA). Fluorescence intensities were quantified and analyzed using the GECOS software (Affymetrix).

**Gene expression profiling and statistical analyses**

Gene expression data were normalized with the MAS5 algorithm and analyzed with inventor's bioinformatics platforms - RAGE (http://rage.montp.inserm.fr/) (35) and Amazonia (http://amazonia.montp.inserm.fr/) (36) - or SAM (Significance Analysis of Microarrays) software (37). The statistical significance of differences in overall survival between groups of patients was calculated by the log-rank test. Multivariate analysis was performed using the Cox proportional hazards model. Survival curves were plotted using the Kaplan-Meier method. All these analyses have been done with R.2.10.1 (http://www.r-project.org/) and bioconductor version 2.5. Gene annotation and networks were generated through the use of Ingenuity Pathways Analysis (Ingenuity® Systems, Redwood City, CA) (38).

## Results

### Modulation of gene expression by decitabine in HMCLs: identification of prognostic genes

Five HMCLs were treated with 0.5 $\mu$M of decitabine for 7 days, a concentration which did not affect myeloma cell viability (Supplementary Table S2) (19). Using SAM supervised paired analysis, the expression of 48 genes was found to be significantly upregulated and that of 79 genes downregulated by decitabine treatment of 5 HMCLs (FDR < 5%; Supplementary Table S3 and S4). Decitabine-regulated genes are significantly enriched in genes related to "Cancer" and "Cell death" pathways (FDR < 5%; Ingenuity pathway analysis. Investigating the expression of these 127 decitabine-regulated genes in primary MMCs of a cohort of 206 newly-diagnosed patients (HM cohort), 22 genes had bad prognostic value and 25 a good one after Benjamini-Hochberg multiple testing correction (Supplementary Table S5). The prognostic information of decitabine regulated genes was gathered within an DNA methylation score (DM Score), which was the sum of the beta coefficients of the Cox model weighted by $\pm$ 1 according to the patient MMC signal above or below the probe set maxstat value as previously described (38). The value of DM Score in normal, premalignant or malignant plasma cells is displayed in Figure 1A. There is no significant difference of DM Score between cells from MGUS patients and normal BMPCs. MMCs of patients had a significantly higher DM Score than normal BMPCs or plasma cells from MGUS-patients ($P$ < .01), and HMCLs the highest score ($P$ < .001) (Figure 1A). Investigating the DM Score in the 8 groups of the molecular classification of multiple myeloma (39), DM Score is significantly higher in the proliferation, t(4;14) and MAF subgroups ($P$ < .001) associated with a poor prognosis (39) and significantly lower in the low bone disease subgroup ($P$ < .001) (39) (Figure 1B).

### Prognostic value of DM score compared to usual prognostic factors

DM Score had prognostic value when used as a continuous variable ($P \leq 10^{-4}$), or by splitting patients into two groups using Maxstat R function (38). A maximum difference in overall survival (OS) was obtained with DM Score = -15.3 splitting patients in a high-risk group of 34.5% patients (DM Score > - 15.3) with a 42.1 months median OS and a low risk group of 65.5% patients (DM Score $\leq$ -15.3) with not reached median survival (Figure 2).

Using univariate Cox analysis, DM Score, UAMS-HRS, IFM-score and GPI had prognostic value as well as t(4;14), del17p, $\beta$2m, albumin and ISS using the HM patient cohort (Supplementary Table S6). When compared two by two, DM Score tested with $\beta$2m remained significant. When these parameters were tested together, DM Score, $\beta$2m and t(4;14) kept prognostic value. The DM Score is also prognostic in an independent cohort of 345 patients from UAMS treated with TT2 therapy (UAMS-TT2 cohort). For each patient of UAMS-TT2 cohort, DM Score was computed using parameters defined with HM patients' cohort. The median OS of patients within high score group (DM Score > - 15.3) was 53.7 months and not reached for patients with low DM Score ($P$ = .0001) (Figure 2). Using Cox univariate analysis, UAMS-HRS, IFM and GPI scores as well t(4;14) and del17p had prognostic value. Comparing these prognostic factors two by two, DM Score remained significant compared to GPI, t(4;14), and del17p in the UAMS-TT2 cohort (Supplementary Table S5). When these parameters were tested together, UAMS-HRS, t(4;14) and del17p kept prognostic value in UAMS-TT2 cohort.

### DM Score is predictive for sensitivity of human myeloma cell lines or patients' primary MMCs to Decitabine *in vitro.*

The inventors sought to determine whether DM Score could predict for the sensitivity of 10 HMCLs to DNMT inhibitor. Starting from a large cohort of 40 HMCLs (22), the 10 HMCLs with the highest or lowest DM Score were selected to assay decitabine sensitivity. The 5 HMCLs with the highest DM Score exhibited a significant 11-fold higher decitabine sensitivity (median IC50 = 0.68 $\mu$M; range: 0.15 to 2.22 $\mu$M) than the 5 HMCLs with low DM Score ($P$ = .01; median IC50 = 7.94 $\mu$M; range: 2.92 to 60.81$\mu$M) (Figure 3). All HMCLs with the lowest DM Score and poorly sensitive to decitabine presented no ras mutations whereas 4 out of 5 HMCLs with the highest DM Score and higher decitabine sensitivity have ras mutations (Table 1).

Primary MMCs were cultured with their BM environment and recombinant IL-6 and graded concentrations of decitabine for 4 days. Primary MMCs of patients with a DM Score above median value (-19.7, Figure 1) exhibited significant ($P$ < .01) 2.2-fold higher decitabine sensitivity than MMCs with DM Score below median (Figure 4 and Table 2). The characteristics of patients with MM included in this study are described in Table 3.

## Discussion

In this study, the inventors have identified a gene expression-based DNA methylation score (DM Score) which is predictive for patients' survival and for the *in vitro* sensitivity of human myeloma cell lines or patients' primary myeloma cells to a DNMT inhibitor, decitabine. Clinical trials in multiple myeloma combining these demethylating agents with chemotherapy or other agents are underway (8). The current identification of DM Score should be very useful to investigate whether the higher response to DNMTi is found in patients with highest DM Score and to speed up the investigation of the clinical efficacy of the novel agents.

Besides the potential utility of the current DM Score in selecting patients who could benefit from DNMTi therapies, the current study highlights pathways which could be involved in the emergence of multiple myeloma cells. Among the genes downregulated by decitabine treatment and associated with a poor prognosis, the inventors identified *RECQ1* (ATP-dependent DNA helicase Q1) and *KIF21B* (kinesin family member 21B). RECQ helicase are a ubiquitous family of DNA unwinding enzymes involved in the maintenance of chromosome stability (40-42). Mutations in the genes of RECQ family members are linked with genetic disorders associated with genomic instability, cancer predisposition and features of premature ageing. Consistent with their ability to unwind DNA, several functions have been attributed to RECQ proteins, including roles in stabilization and repair of damaged DNA replication forks, telomere maintenance, homologous recombination, and DNA damage checkpoint signaling (40-42). Recent reports supported a cancer specific role for RECQ1 (43-45). RECQ1 silencing in cancer cells resulted in mitotic catastrophe and administration of siRNA targeting RECQ1 prevented tumor growth in murine models (43-45). More recently, it was demonstrated that RECQ1 is highly expressed in various types of solid tumors including colon carcinoma, thyroid cancer, lung cancer and brain glioblastoma tissues (46) In glioblastoma cell lines, depletion of RECQ1 by RNAi results in a significant reduction of cellular proliferation, perturbation of S-phase progression, spontaneous $\gamma$-H2AX foci formation and hypersensitivity to hydroxyurea and temozolomide treatments (46). KIF21B is a kinesin family member. Kinesins are a conserved class of microtubule-dependent molecular motor proteins that have adenosine triphosphatase activity and motion characteristics (47). Kinesins support several cellular functions, such as mitosis, meiosis and the transport of macromolecules. In mitosis of eukaryotic cells, kinesins participate in spindle formation, chromosome congression and alignment, and cytokinesis (48). Abnormal expression and function of kinesins are involved in the development or progression of several kinds of human cancers (49,50). Interestingly, KIF21B maps to chromosome 1q arm (1q32.1) whose amplification characterizes a significant fraction of high-risk MM tumors (51). More recently, *KIF21B* gene was found in a critical neighbor-gene model associated with a poor prognosis across independent data sets of respectively, 559, 247 and 264 myeloma patients (52). These data suggest that decitabine treatment could synergize with DNA-damaging agents, in MM, targeting genes involved in DNA-repair and maintenance of chromosome stability. These results demonstrated that the study of MMCs response to epigenetic-targeted treatments will extend our knowledge of MM development and progression and will lead to potential therapeutic advances. Epigenetic therapies could be combined with conventional therapies to develop personalized treatments in MM, render resistant tumors responsive to treatment.

Table 1: Characteristics of HMCLs[5-aza sensitive] and HMCLs[5-aza-resistant]

| HMCL Name | IL-6 dependence[1] | Origin[2] | Disease[3] | Patient sample[4] | Gender | Isotype | t(14q32 or 22q11) | Target genes | Ras | TP53 | CD45 | HMCL classification |
|---|---|---|---|---|---|---|---|---|---|---|---|---|
| **5-aza Resistant HMCLs** | | | | | | | | | | | | |
| XG6 | ++ | MN | MM | PB | F | GI | t(16;22) | *c-Maf* | *wt* | *wt* | + | CTA/MF |
| XG20 | ++ | MN | PCL | PB | M | I | t(4;14) | *MMSET* | *wt* | *abn* | - | MS |
| XG13 | ++ | MN | PCL | PB | M | GI | t(14;16) | *c-Maf* | *wt* | *abn* | + | MF |
| SKMM2 | - | CO | PCL | PB | M | Gk | t(11;14) | *CCND1* | *wt* | *abn* | - | CD-1 |
| LP1 | - | CO | MM | PB | F | GI | t(4;14) | *MMSET/ FGFR3* | *wt* | *abn* | - | MS |
| **5-aza Sensitive HMCLs** | | | | | | | | | | | | |
| XG12 | ++ | MN | PCL | PB | F | I | t(14;16) | *c-Maf* | *mut* | *wt* | + | CTA/MF |
| XG16 | ++ | MN | PCL | PB | M | k | none | *none* | *mut* | *abn* | + | CTA/FRZB |
| XG19 | ++ | MN | PCL | PB | F | AI | t(14;16) | *c-Maf* | *wt* | *wt* | + | CTA/MF |
| JJN3 | - | CO | MM | PE | F | Ak | t(14;16) | *c-Maf* | *mut* | *abn* | +/- | MF |
| RPMI8226 | - | CO | MM | PB | M | GI | t(14;16) | *c-Maf* | *mut* | *abn* | - | MF |

[1]++ if growth is strictly dependent on adding exogenous IL-6, + if dependent on adding exogenous IL-6 - if not; [2]Origin of the HMCL, MN Montpellier or Nantes, CO collected; [3]Disease at diagnosis: MM multiple myeloma, PCL plasma celle leukemia, PCT plasmacytoma; [4]Origin of the sample: AF ascitic fluid, BM bone marrow, PE pleural effusion, PB peripheral blood.

**Table 2:** Mononuclear cells from tumor samples of 12 patients with MM were cultured for 4 days in the presence of IL-6 (2 ng/mL) with or without increased doses of decitabine. At day 4 of culture, the cell count and viability were determined and the percentage of CD138$^+$ viable plasma cells was determined by flow cytometry.

| | | Myeloma cell number/culture well | | | | |
|---|---|---|---|---|---|---|
| | Patient no. | Control | 0.125µM decitabine | 0.5µM decitabine | 2µM decitabine | 8µM decitabine |
| **Patients with high DM Score** | 1 | 72168 | 43320 | 36738 | 20592 | 5800 |
| | 2 | 78182 | 49096 | 31086 | 22880 | 11600 |
| | 3 | 60140 | 40432 | 28260 | 18304 | 10150 |
| | 4 | 108252 | 63536 | 56520 | 29744 | 18850 |
| | 5 | 117273 | 69312 | 42390 | 32032 | 17400 |
| | 6 | 96224 | 60648 | 36738 | 20592 | 13050 |
| | Mean | 88707 | 54391 | 38622 | 24024 | 12808 |
| **Patients with low DM Score** | 1 | 24880 | 26100 | 27195 | 21028 | 18265 |
| | 2 | 57133 | 51984 | 45216 | 32032 | 21750 |
| | 3 | 132308 | 77976 | 53694 | 22880 | 14500 |
| | 4 | 53664 | 54336 | 51300 | 51230 | 53486 |
| | 5 | 1068 | 1066 | 870 | 1044 | 1039 |
| | 6 | 23716 | 51024 | 54726 | 55818 | 47440 |
| | Mean | 48795 | 43748 | 38834 | 30672 | 26080 |

**Table 3:** Characteristics of patients with a DM Score above (N = 6) and under (N = 6) the median value.

| | Age | Sex | Monoclonal protein | Durie and Salmon stage | ISS stage | Serum β2-microglobulin | Multiple myeloma molecular classification |
|---|---|---|---|---|---|---|---|
| **Patients with high DM Score** | | | | | | | |
| Patient 1 | 59 | F | IgA Lambda | IIIA | III | 6,7 | MF |
| Patient 2 | 48 | M | Lambda | NA | NA | NA | CD1 |
| Patient 3 | 63 | M | BJ Lambda | NA | NA | NA | PR |
| Patient 4 | 69 | M | IgG Lambda | IIIA | III | 10.4 | PR |
| Patient 5 | 70 | F | IgA Lambda | IIIA | II | 5 | CD2 |
| Patient 6 | 63 | F | Asecret | III | III | 13.5 | CD1 |
| **Patients with low DM Score** | | | | | | | |
| Patient 1 | 72 | M | IgG Lambda | IIIA | III | 8.6 | PR |
| Patient 2 | 54 | M | IgA Lambda | IIIA | I | 2.3 | CD2 |
| Patient 3 | 62 | M | IgA Kappa | IIA | I | 2.6 | HY |
| Patient 4 | 55 | M | BJ Kappa | IIIB | III | 10 | HY |
| Patient 5 | 69 | F | IgG Kappa | IIIA | I | 2.8 | CD2 |
| Patient 6 | 61 | M | BJ Kappa | IIIA | II | 4.6 | HY |

**Supplementary Table S1. Clinical patient data for age, serum-β2-microglobulin, and plasma cell infiltration** in the Heidelberg/Montpellier-group (HM) and the Arkansas cohort. Median value and range are given. NA, not available. ISS, International Staging System.

| Characteristic | HM cohort (n=206) | Arkansas cohort (n=345) |
|---|---|---|
| Age | 58,5 [27 - 73] | 57 [25 - 77] |
| **Monoclonal protein** | | |
| IgG | 120 | 193 |
| IgA | 46 | 93 |
| Bence Jones | 35 | 47 |
| Asecretory | 4 | 6 |
| IgD | 1 | 3 |
| NA | 0 | 3 |
| **Myeloma in Durie and Salmon stage** | | |
| I | 22 | NA |
| II | 31 | NA |
| III | 153 | NA |
| Myeloma in ISS stage | | |
| I | 97 | 189 |
| II | 73 | 86 |
| III | 33 | 70 |
| NA | 3 | 0 |
| Serum-β2-microglobulin | 2.99 [1.3 - 53.6] | 2.9 [1.0 - 38.7] |
| Plasma cells in bone marrow | 42 [1 - 100] | 42 [4 - 98] |

**Supplementary Table S2: Cell viability of HMCLs treated with either 0.5 μM decitabine for 7 days.** Date are the mean percentages ± SD of viable cells evaluated by trypan blue exclusion (3 experiments).

| | Cell viability (%) | | | | |
|---|---|---|---|---|---|
| | Day 0 | Day 3 | | Day 7 | |
| HMCLS | | Control | 5-aza | Control | 5-aza |
| XG-5 | 70±2 | 70±1 | 65±5 | 83±5 | 69±6 |
| XG-6 | 90±2 | 90±2 | 90±1 | 93±5 | 90±1 |
| XG-7 | 100±0 | 90±2 | 90±2 | 93±5 | 85±5 |
| XG-20 | 100±0 | 91±3 | 91±3 | 95±5 | 83±5 |
| LP1 | 100±0 | 91±2 | 91±2 | 95±5 | 86±4 |

**Supplementary Table S3:** Genes overexpressed in decitabine treated HMCLs. Five HMCLs were cultured with or without 0.5 μM decitabine for 7 days and gene expression was profiled with Affymetrix U133 plus 2.0. Genes significantly differentially expressed between control and decitabine treated cells were identified using SAM supervised paired analysis with a 5% false discovery rate. When a gene was interrogated by several probe sets, we used the probe set yielding to a maximum variance across control and decitabine treated cells.

| Probeset | Gene | Ratio | Banding | Affymetrix description |
|---|---|---|---|---|
| **Intercellular communication and membrane proteins** | | | | |
| 209122_at | ADFP | 3.70 | 9p22.1 | adipose differentiation-related protein |
| 211990_at | HLA-DPA1 | 1.70 | 6p21.3 | major histocompatibility complex; class II; DP alpha 1 |
| 205718_at | ITGB7 | 2.11 | 12q13.13 | integrin; beta 7 |
| 1569003_at | TMEM49 | 2.33 | 17q23.2 | transmembrane protein 49 |
| 205483_s_at | G1P2 | 2.77 | 1p36.33 | interferon; alpha-inducible protein (clone IFI-15K) |
| 200696_s_at | GSN | 3.97 | 9q33 | gelsolin (amyloidosis; Finnish type) |
| **Signal transduction** | | | | |
| 203964_at | NMI | 1.86 | 2p24.3-q21.3 | N-myc (and STAT) interactor |
| 205552_s_at | OAS1 | 1.65 | 12q24.1 | 2prime;5prime-oligoadenylate synthetase 1; 40/46kDa |
| 209969_s_at | STAT1 | 2.55 | 2q32.2 | signal transducer and activator of transcription 1; 91kDa |
| 202693_s_at | STK17A | 2.52 | 7p12-p14 | serine/threonine kinase 17a (apoptosis-inducing) |
| **Cytoskeleton** | | | | |
| 223129_x_at | MYLIP | 2.00 | 6p23-p22.3 | myosin regulatory light chain interacting protein |
| 209083_at | CORO1A | 2.38 | 16p11.2 | coronin; actin binding protein; 1A |
| 216323_x_at | H2-ALPHA | 2.56 | 2q21.1 | alpha-tubulin isotype H2-alpha |
| 210527_x_at | TUBA2 | 2.50 | 13q11 | tubulin; alpha 2 |
| 209118_s_at | TUBA3 | 2.23 | 12q12-12q14.3 | tubulin; alpha 3 |
| 204141_at | TUBB2 | 5.07 | 6p25 | tubulin; beta 2 |
| **Cancer testis antigens** | | | | |
| 235700_at | CT45-2 | 30.39 | Xq26.3 | cancer/testis antigen CT45-2 |
| 210437_at | MAGEA 9 | 1.68 | Xq28 | melanoma antigen family A; 9 |
| 207847_s_at | MUC1 | 2.92 | 1q21 | mucin 1; transmembrane |
| **Protein binding** | | | | |
| 202411_at | IFI27 | 2.84 | 14q32 | interferon; alpha-inducible protein 27 |
| 224917_at | MIRN21 | 1.99 | --- | microRNA 21 |
| 202814_s_at | HEXIM1 | 2.18 | 17q21.31 | hexamethylene bis-acetamide inducible 1 |
| 211071_s_at | MLLT 11 | 2.53 | 1q21 | myeloid/lymphoid or mixed-lineage leukemia (trithorax homolog; Drosophila); translocated to 11 |

(continued)

| Protein binding | | | | |
|---|---|---|---|---|
| 33322_ i_at | SFN | 4.34 | 1p36.11 | stratifin |
| **Metabolism** | | | | |
| 207761_s_at | DKFZP5 86A0522 | 2.97 | 12q13.12 | DKFZP586A0522 protein / METTL7A methyltransferase like 7A |
| 201468_s_at | NQO1 | 2.15 | 16q22.1 | NAD(P)H dehydrogenase; quinone 1 |
| 218543_s_at | PARP12 | 1.92 | 7q34 | poly (ADP-ribose) polymerase family; member 12 |
| 214183_s_at | TKTL1 | 85.24 | Xq28 | transketolase-like 1 |
| 204079_at | TPST2 | 2.20 | 22q12.1 | tyrosylprotein sulfotransferase 2 |
| 201243_s_at | ATP1B1 | 3.43 | 1q24 | ATPase; Na+/K+ transporting; beta 1 polypeptide |
| 210580_x_at | SULT1A 3 | 2.52 | 16p11.2 | sulfotransferase family; cytosolic; 1A; phenol-preferring; member 3 |
| **Nuclear proteins and transcription factors** | | | | |
| 238825_at | ACRC | 25.99 | Xq13.1 | acidic repeat containing |
| 31845_at | ELF4 | 1.75 | Xq26 | E74-like factor 4 (ets domain transcription factor) |
| 208012_x_at | SP110 | 2.11 | 2q37.1 | SP 110 nuclear body protein |
| 210387_at | HIST1H2 BG | 2.00 | 6p21.3 | histone 1; H2bg |
| 201565_s_at | ID2 | 2.76 | 2p25 | inhibitor of DNA binding 2; dominant negative helix-loop-helix protein |
| 223484_at | NMES1 | 23.96 | 15q21.1 | normal mucosa of esophagus specific 1 |
| **Apoptosis** | | | | |
| 1552701_a_a t | COP1 | 2.34 | --- | caspase-1 dominant-negative inhibitor pseudo-ICE |
| 209417_s_at | IFI35 | 1.93 | 17q21 | interferon-induced protein 35 |
| 202086_at | MX1 | 1.73 | 21q22.3 | myxovirus (influenza virus) resistance 1; interferon-inducible protein p78 (mouse) |
| 219099_at | C12orf5 | 2.16 | 12p13.3 | chromosome 12 open reading frame 5 |
| 201631_s_at | IER3 | 3.97 | 6p21.3 | immediate early response 3 |
| **Others** | | | | |
| 227609_at | EPSTI1 | 1.99 | 13q13.3 | epithelial stromal interaction 1 (breast) |
| 217755 at | HN1 | 2.17 | 17q25.1 | hematological and neurological expressed 1 |
| 215343_at | KIAA1509 | 2.21 | 14q32.12 | KIAA1509 |
| 228531_at | SAMD9 | 2.06 | 7q21.2 | sterile alpha motif domain containing 9 |
| 230000_at | C17orf27 | 2.41 | 17q25.3 | chromosome 17 open reading frame 27 |

(continued)

| Others | | | | |
|---|---|---|---|---|
| 235964_x_at | C20orf1 18 | 2.12 | 20 | Chromosome 20 open reading frame 118 |

**Supplementary Table S4:** Genes underexpressed in decitabine treated HMCLs. Five HMCLs were cultured with or without 0.5 μM decitabine for 7 days and gene expression was profiled with Affymetrix U133 plus 2.0. Genes significantly differentially expressed between control and decitabine treated cells were identified using SAM supervised paired analysis with a 5% false discovery rate. When a gene was interrogated by several probe sets, we used the probe set yielding to a maximum variance across control and decitabine treated cells.

| Probeset | Gene | Ratio | Banding | Affymetrix description |
|---|---|---|---|---|
| **Intercellular communication and membrane proteins** | | | | |
| 202304_at | FNDC3A | 0.40 | 13q14.2 | fibronectin type III domain containing 3A |
| 209541_at | IGF1 | 0.43 | 12q22-q23 | insulin-like growth factor 1 (somatomedin C) |
| 221658_s_at | IL21R | 0.50 | 16p11 | inter leukin 21 receptor |
| 210587_at | INHBE | 0.55 | 12q13.3 | inhibin; beta E |
| 219702_at | PLAC1 | 0.42 | Xq26 | placenta-specific 1 |
| 209606_at | PSCDBP | 0.48 | 2q11.2 | pleckstrin homology; Sec7 and coiled-coil domains; binding protein |
| 202375_at | SEC24D | 0.31 | 4q26 | SEC24 related gene family; member D (S. cerevisiae) |
| 222838_at | SLAMF7 | 0.51 | 1q23.1-q24.1 | SLAM family member 7 |
| 222690_s_at | TMEM39A | 0.46 | 3q13.33 | transmembrane protein 39A |
| **Signal transduction** | | | | |
| 224451_x_at | ARHGAP9 | 0.38 | 12q14 | Rho GTPase activating protein 9 /// Rho GTPase activating protein 9 |
| 212954_at | DYRK4 | 0.42 | 12p13.32 | dual-specificity tyrosine-(Y)-phosphorylation regulated kinase 4 |
| 228831_s_at | GNG7 | 0.33 | 19p13.3 | guanine nucleotide binding protein (G protein); gamma 7 |
| 209314_s_at | HBS1L | 0.53 | 6q23-q24 | HBS1-like (S. cerevisiae) |
| 219188_s_at | LRP16 | 0.36 | 11q11 | LRP16 protein |
| 229549_at | OPN1SW | 0.40 | 7q31.3-q32 | Opsin 1 (cone pigments); short-wave-sensitive (color blindness; tritan) |
| **Cytoskeleton** | | | | |
| 213500_at | COPB2 | 0.58 | 3q23 | Coatomer protein complex; subunit beta 2 (beta prime) |
| 204411_at | KIF21B | 0.53 | 1pter-q31.3 | kinesin family member 21B |
| 226438_at | SNTB1 | 0.36 | 8q23-q24 | Syntrophin; beta 1 (dystrophin-associated protein A1; 59kDa; basic component 1) |
| 226181_at | TUBE1 | 0.38 | 6q21 | tubulin; epsilon 1 |
| Cell cycle | | | | |
| 214152_at | CCPG1 | 0.44 | 15q21.1 | cell cycle progression 1 |

(continued)

| Cell cycle | | | | |
|---|---|---|---|---|
| 223569_at | PPAPDC1B | 0.42 | 8p12 | phosphatidic acid phosphatase type 2 domain containing 1B |
| 223195_s_at | SESN2 | 0.47 | 1p35.3 | sestrin 2 |
| **Metabolism** | | | | |
| 231202_at | ALDH1L2 | 0.45 | 12q23.3 | aldehyde dehydrogenase 1 family; member L2 |
| 219572_at | CADPS2 | 0.37 | --- | Ca2+-dependent activator protein for secretion 2 |
| 212816_s_at | CBS | 0.41 | 21q22.3 | cystathionine-beta-synthase |
| 218923_at | CTBS | 0.42 | 1p22 | chitobiase; di-N-acetyl- |
| 201791_s_at | DHCR7 | 0.45 | 1 1q13.2-q13.5 | 7-dehydrocholesterol reductase |
| 204646_at | DPYD | 0.48 | 1p22 | dihydropyrimidine dehydrogenase |
| 203397_s_at | GALNT3 | 0.65 | 2q24-q31 | UDP-N-acetyl-alpha-D-galactosamine: polypeptide N-acetylgalactosaminyltransferase 3 (GalNAc-T3) |
| 203157_s_at | GLS | 0.42 | 2q32-q34 | glutaminase |
| 226183_at | GSK3B | 0.49 | 3q13.3 | Glycogen synthase kinase 3 beta |
| 1555037_a_at | IDH1 | 0.53 | 2q33.3 | isocitrate dehydrogenase 1 (NADP+); soluble |
| 201625_s_at | INSIG1 | 0.34 | 7q36 | insulin induced gene 1 |
| 221760_at | MAN1A1 | 0.43 | 6q22 | Mannosidase; alpha; class 1A; member 1 |
| 222805_at | MANEA | 0.51 | 6q16.1 | mannosidase; endo-alpha |
| 232488_at | MGC15875 | 0.55 | 5q35.3 | hypothetical protein MGC15875 |
| 225520_at | MTHFD1L | 0.41 | 6q25.1 | methylenetetrahydrofolate dehydrogenase (NADP+ dependent) 1-like |
| 202847_at | PCK2 | 0.47 | 14q11.2 | phosphoenolpyruvate carboxykinase 2 (mitochondrial) |
| 221788_at | PGM3 | 0.49 | 6q14.1-q15 | Phosphoglucomutase 3 |
| 201397_at | PHGDH | 0.36 | 1p12 | phosphoglycerate dehydrogenase |
| 205194_at | PSPH | 0.38 | 7p15.2-p15.1 | phosphoserine phosphatase |
| 200831_s_at | SCD | 0.39 | 10q23-q24 | stearoyl-CoA desaturase (delta-9-desaturase) |
| 209610_s_at | SLC1A4 | 0.54 | 2p15-p13 | solute carrier family 1 (glutamate/neutral amino acid transporter); member 4 |
| 208916_at | SLC1A5 | 0.40 | 19q13.3 | solute carrier family 1 (neutral amino acid transporter); member 5 |
| 209921_at | SLC7A11 | 0.41 | 4q28-q32 | solute carrier family 7; (cationic amino acid transporter; y+ system) member 11 |
| **Protein binding** | | | | |
| 215930_s_at | CTAGE5 | 0.30 | 14q13.3 | CTAGE family; member 5 |
| 1554462_a_at | DNAJB9 | 0.42 | 7q31\|14q24 .2-q24.3 | DnaJ (Hsp40) homolog; subfamily B; member 9 |
| 222294_s_at | EIF2C2 | 0.52 | 8q24 | Eukaryotic translation initiation factor 2C; 2 |
| 235745_at | ERN1 | 0.35 | 17q24.2 | endoplasmic reticulum to nucleus signalling 1 |

(continued)

| Protein binding | | | | |
|---|---|---|---|---|
| 219118_at | FKBP11 | 0.38 | 12q13.12 | FK506 binding protein 11; 19 kDa |
| 218361_at | GOLPH3L | 0.45 | 1q21.2 | golgi phosphoprotein 3-like |
| 224763_at | RPL37 | 0.35 | 5p13 | ribosomal protein L37 |
| 201915_at | SEC63 | 0.51 | 6q21 | SEC63-like (S. cerevisiae) |
| 202061_s_at | SEL1L | 0.56 | 14q24.3-q31 | sel-1 suppressor of lin-12-like (C. elegans) |
| 217790_s_at | SSR3 | 0.40 | 3q25.31 | signal sequence receptor; gamma (translocon-associated protein gamma) |
| 202558_s_at | STCH | 0.47 | 21q11.1\|21q11 | stress 70 protein chaperone; microsome-associated; 60kDa |
| 222116_s_at | TBC1D16 | 0.70 | 17q25.3 | TBC1 domain family; member 16 |
| 218145_at | TRIB3 | 0.41 | 20p13-p12.2 | tribbles homolog 3 (Drosophila) |
| Nuclear proteins and transcription factors | | | | |
| 227558_at | CBX4 | 0.49 | 17q25.3 | chromobox homolog 4 (Pc class homolog; Drosophila) |
| 219551_at | EAF2 | 0.46 | 3q13.33 | ELL associated factor 2 |
| 226982_at | ELL2 | 0.45 | 5q15 | elongation factor; RNA polymerase II; 2 |
| 202146_at | IFRD1 | 0.51 | 7q22-q31 | interferon-related developmental regulator 1 |
| 214179_s_at | NFE2L1 | 0.59 | 17q21.3 | nuclear factor (erythroid-derived 2)-like 1 |
| 213878_at | RECQL | 0.44 | 12p12 | RecQ protein-like (DNA helicase Q1-like) |
| 208763_s_at | TSC22D3 | 0.53 | Xq22.3 | TSC22 domain family; member 3 |
| 225382_at | ZNF275 | 0.35 | Xq28 | zinc finger protein 275 |
| 227132_at | ZNF706 | 0.49 | 8q22.3 | zinc finger protein 706 |
| Others | | | | |
| 227755_at | --- | 0.53 | --- | CDNA FLJ42435 fis; clone BLADE2006849 |
| 208725_at | EIF2S2 | 0.46 | --- | eukaryotic translation initiation factor 2, subunit 2 beta, Full-length cDNA clone CS0DD001YD20 of Neuroblastoma Cot 50-normalized of Homo sapiens (human) |
| 244623_at | --- | 0.39 | --- | Transcribed locus |
| 226719_at | --- | 0.53 | --- | CDNA FLJ34899 fis; clone NT2NE2018594 |
| 230570_at | --- | 0.37 | --- | Transcribed locus |
| 229969_at | --- | 0.41 | --- | Transcribed locus; moderately similar to XP_508230.1 PREDICTED: zinc finger protein 195 [Pan troglodytes] |
| 223136_at | AIG1 | 0.41 | 6q24.2 | androgen-induced 1 |
| 222545_s_at | C10orf57 | 0.56 | 10q22.3 | chromosome 10 open reading frame 57 |
| 220755_s_at | C6orf48 | 0.49 | 6p21.3 | chromosome 6 open reading frame 48 |
| 219802_at | FLJ22028 | 0.44 | 12p12.1 | hypothetical protein FLJ22028 |
| 212633_at | KIAA0776 | 0.49 | 6q16.1 | KIAA0776 |
| 229090_at | LOC220930 | 0.45 | 10p11.23 | hypothetical protein LOC220930 |

**Supplementary Table S5:** Prognostic value of decitabine deregulated genes in primary MMC of newly-diagnosed patients

| Probeset | NAME | Ajusted P value | Hazard ratio |
|---|---|---|---|
| **Bad prognostic genes** | | | |
| 209417_s_at | IFI35 | .03 | 1.84 |
| 221658_s_at | IL21R | .04 | 1.84 |
| 1552701_a_at | COP1 | .02 | 1.95 |
| 223129 x at | MYLIP | .01 | 1.99 |
| 214179_s_at | NFE2L1 | .04 | 2.00 |
| 204079_at | TPST2 | .03 | 2.01 |
| 205483_s_at | G1P2 | .01 | 2.02 |
| 33322_i_at | SFN | .03 | 2.05 |
| 222690_s_at | TMEM39A | .02 | 2.20 |
| 209118_s_at | TUBA3 | .01 | 2.21 |
| 208012_ x_at | SP110 | .01 | 2.22 |
| 213878_at | RECQL | .01 | 2.22 |
| 228531_at | SAMD9 | .005 | 2.25 |
| 203397_s_at | GALNT3 | .004 | 2.31 |
| 209969_s_at | STAT1 | .003 | 2.37 |
| 202411_at | IFI27 | .006 | 2.39 |
| 201625_s_at | INSIG1 | .02 | 2.50 |
| 205552_s_at | OAS1 | .01 | 2.50 |
| 204411_at | KIF21B | .0005 | 2.88 |
| 209083_at | CORO1A | .00008 | 3.05 |
| 218543_s_at | PARP12 | .00005 | 3.52 |
| 205718_at | ITGB7 | .00001 | 4.03 |
| Good prognostic genes | | | |
| 221788_at | PGM3 | .0003 | .29 |
| 229969_at | SEC63 | .0003 | .29 |
| 224763_at | RPL37 | .0006 | .32 |
| 232488_at | MGC15875 | .0007 | .35 |
| 202558_s_at | STCH | .04 | .39 |
| 219118_at | FKBP 11 | .03 | .41 |
| 223569_at | PPAPDC1B | .01 | .42 |
| 220755_s_at | C6orf48 | .04 | .43 |
| 202304_at | FNDC3A | .009 | .44 |
| 1554462_a_at | DNAJB9 | .02 | .45 |
| 227755_at | EST, GenBank AA042983 | .01 | .45 |
| 226183_at | GSK3B | .02 | .46 |
| 209122_at | ADFP | .01 | .47 |

(continued)

| Good prognostic genes | | | |
|---|---|---|---|
| 219551_at | EAF2 | .03 | .47 |
| 224451_x_at | ARHGAP9 | .03 | .48 |
| 210387_at | HIST1H2BG | .01 | .49 |
| 211990_at | HLA-DPA1 | .01 | .49 |
| 202061_s_at | SEL1L | .04 | .50 |
| 214152_at | CCPG1 | .02 | .50 |
| 226982_at | ELL2 | .03 | .50 |
| 222294_s_at | EIF2C2 | .02 | .52 |
| 228831_s_at | GNG7 | .04 | .52 |
| 230570_at | EST, GenBank AI702465 | .04 | .52 |
| 208725_at | EIF2S2 | .04 | .54 |
| 222838_at | SLAMF7 | .04 | .55 |

**Supplementary Table S6: Cox univariate and multivariate analysis of OS in HM and TT2 patients' cohorts.**

| | | HM Cohort | | TT2 Cohort | |
|---|---|---|---|---|---|
| | | OAS | | OAS | |
| | Pronostic variable | Proportional hazard ratio | *P*-value | Proportional hazard ratio | *P*-value |
| Univariate COX analysis - Overall survival | DM Score | 7.12 | <.0001 | 2.06 | <.0001 |
| | $\beta$2m | 1.1 | <.0001 | NA | NA |
| | ISS | 1.73 | .001 | NA | NA |
| | HRS | 2.37 | .01 | 4.67 | <.0001 |
| | IFM score | 3.09 | .0001 | 1.78 | .004 |
| | t(4 ;14) | 2.14 | .001 | 2.21 | .001 |
| | del17p | 3.44 | .02 | 2.46 | <.0001 |
| | GPI | 2.21 | .0001 | 1.75 | <.0001 |

(continued)

|  | | HM Cohort | | TT2 Cohort | |
|  | | OAS | | OAS | |
|  | Pronostic variable | Proportional hazard ratio | P-value | Proportional hazard ratio | P-value |
|---|---|---|---|---|---|
| Multivariate COX analysis - Overall survival | DM Score<br>ISS | 5.91<br>1.40 | <.0001<br>NS | NA<br>NA | NA<br>NA |
|  | DM Score<br>β2m | 6.31<br>1.1 | <.0001<br>.017 | NA<br>NA | NA<br>NA |
|  | DM Score<br>HRS | 6.68<br>1.47 | <.0001<br>NS | 1.46<br>4.02 | NS<br><.0001 |
|  | DM Score<br>IFM score | 6.80<br>1.33 | <.0001<br>NS | 1.81<br>1.37 | .006<br>NS |
|  | DM Score<br>t(4 ;14) | 6.28<br>1,88 | <.0001<br>NS | 1.94<br>2.01 | .001<br>.003 |
|  | DM Score<br>del17p | 6.34<br>1.60 | <.0001<br>NS | 2.00<br>2.32 | <.0001<br>.001 |
|  | DM Score<br>GPI | 5.97<br>1.47 | <.0001<br>NS | 2.00<br>1.68 | <.0001<br><.0001 |
| Multivariate COX analysis - Overall survival | DM Score<br>β2m<br>ISS<br>HRS<br>IFM score<br>t(4 ;14)<br>del17p<br>GPI | 7.84<br>1.1<br>1.37<br>1.28<br>.62<br>2.58<br>1.72<br>1.61 | .005<br>NS<br>NS<br>NS<br>NS<br>.02<br>NS<br>NS | 1.40<br>NA<br>NA<br>3.66<br>.34<br>2.36<br>2.21<br>1.11 | NS<br>NA<br>NA<br><.0001<br>NS<br><.0001<br>.002<br>NS |

The prognostic factors were tested as single variable or multi variables using Cox-model. P-values and the hazard ratios (HR) are shown. NS, Not significant at a 5% threshold; GPI, gene expression based proliferation index; ISS, International Staging System; HRS, high-risk score; IFM, Intergroupe Francophone du Myélome; NA, Not available.

**EXAMPLE 2:**

In order to identify the minimal number of genes among the 47 genes used to calculate the DM score, the inventors used PAM (Prediction Analysis of Microarray) statistical technique used for class prediction from gene expression data using nearest shrunken centroids. 25 genes were identified and are depicted in Table B.

Table B:

| Probesets | Name |
|---|---|
| 202061_s_at | SEL1L |
| 202304_at | FNDC3A |
| 202558_s_at | STCH |
| 203397_s_at | GALNT3 |
| 204079_at | TPST2 |
| 205483_s_at | G1P2 |
| 205552_s_at | OAS1 |

(continued)

| Probesets | Name |
|---|---|
| 205718_at | ITGB7 |
| 208725_at | EIF2S2 |
| 209083_at | CORO1A |
| 209118_s_at | TUBA3 |
| 209122_at | ADFP |
| 209417_s_at | IFI35 |
| 211990_at | HLA-DPA1 |
| 214152_at | CCPG1 |
| 214179_s_at | NFE2L1 |
| 219118_at | FKBP11 |
| 221658_s_at | IL21R |
| 222690_s_at | TMEM39A |
| 223129_x_at | MYLIP |
| 224451_x_at | ARHGAP9 |
| 224763_at | RPL37 |
| 227755_at | --- |
| 228831_s_at | GNG7 |
| 232488_at | MGC15875 |

**EXAMPLE 3: DNA methylation score is predictive of myeloma cell sensitivity to 5-azacitidine.**

Epigenetics is characterized by a wide range of changes that are reversible and orchestrate gene expression. Recent studies of the epigenome have shown that epigenetic modifications play a role in cancer physiopathology including hematologic malignancies (Issa 2007, Issa, et al 2004, Oki, et al 2008, Smith, et al 2009). In MM, DNA hypomethylation was reported as the predominant early change during myelomagenesis that is gradually transformed to DNA hypermethylation in relapsed cases and during the progression of the disease (Heuck, et al 2013, Walker, et al 2010). Hypermethylation of GPX3, RBP1, SPARC and TGFBI genes was demonstrated to be associated with significantly shorter overall survival, independent of age, ISS score and adverse cytogenetics (Kaiser, et al 2013). DNA methylation is regulated by DNA methyltransferases (DNMT) (Hollenbach, et al 2010). Decitabine (5-aza-2'-deoxycytidine) or 5-azacytidine are both FDA (U.S. Food and Drug Administration)-approved DNMT inhibitors for the treatment of myelodysplastic syndrome (MDS) (Rodriguez-Paredes and Esteller 2011). 5-azacytidine is a ribonucleoside and Decitabine is a deoxy-yribonucleoside. Decitabine is incorporated only in DNA whereas 5-azacytidine incorporates both in DNA and RNA including rRNAs, tRNAs, mRNAs and miRNAs (Hollenbach, et al 2010). Once incorporated into DNA, 5-azacytidine and Decitabine will lead to DNMTs depletion, DNA hypomethylation and DNA damage induction (Flotho, et al 2009, Ghoshal, et al 2005, Hollenbach, et al 2010, Kiziltepe, et al 2007, Palii, et al 2008, Stresemann, et al 2006). Via incorporation into newly synthetized RNA, 5-azacytidine will alterate the processing of RNAs inhibiting protein synthesis (Hollenbach, et al 2010). The inventors have recently reported the building of a DNA methylation score (DM Score) predicting for the efficacy of decitabine to kill MM cells (MMCs). Given that 5-azacitidine can incorporate in both DNA and RNA and block DNA methylation and RNA traduction, The inventors have currently investigated whether DM Score could also predict for MMCs sensitivity to 5-azacitidine.

**Material & Methods**

**Human Myeloma Cell Lines (HMCLs) and primary multiple myeloma cells of patients.**

Human myeloma cell lines XG-1, XG-2, XG-5, XG-6, XG-12, XG-13, XG-16, XG-19, XG-20, RPMI8226, LP1 and SKMM2 (HMCLs, N=12) were obtained as previously described (Gu, et al 2000, Moreaux, et al 2011, Rebouissou, et al 1998, Tarte, et al 1999, Zhang, et al 1994) or purchased from DSMZ and American Type Culture Collection. These HMCLs were extensively phenotypically and molecularly characterized as described (Moreaux, et al 2011). Microarray data are deposited in the ArrayExpress public database (accession numbers E-TABM-937 and E-TABM-1088). Bone marrow of patients presenting with previously untreated multiple myeloma (n=14) at the university hospital of Montpellier were obtained after patients' written informed consent in accordance with the Declaration of Helsinki and agreement of the Montpellier University Hospital Center for Biological Resources. MMCs were purified as previously published (Mahtouk, et al 2004) and whole genome gene expression profiling assayed with Affymetrix U133 2.0 plus microarrays (Affymetrix, Santa Clara, CA, USA) (De Vos, et al 2002). Gene expression data were analyzed using the inventor's bioinformatics platforms (http://rage.montp.inserm.fr/ and http://amazonia.montp.inserm.fr/) (Reme, et al 2008, Tanguy Le Carrour 2010) and computations performed using R 2.15.1 (http://www.r-project.org/) and bioconductor 2.0 (http://www.bioconductor.org).

**Sensitivity of myeloma cell lines and primary myeloma cells to 5-azacitidine.**

HMCLs were cultured with graded 5-azacitidine (Sigma, St Louis, MO) concentrations. HMCLs cell growth was quantified with a Cell Titer Glo Luminescent Assay (Promega, Madison, WI) and half inhibitory concentration (IC50) was determined using GraphPad Prism (http://www.graphpad.com/scientific-software/prism/).

Primary myeloma cells of 14 patients were cultured with or without graded concentrations of 5-azacitidine and MMC cytotoxicity evaluated using anti-CD138-PE mAb (Immunotech, Marseille, France) as described (Jourdan, et al 1998; Mahtouk, et al 2004; Moreaux, et al 2012).

**Gene set enrichment analysis (GSEA)**

The inventors compared the gene expression levels from high DM Score versus low DM Score patients and picked up the genes which had significant different expression for Gene set enrichment analysis (GSEA). Gene set enrichment analysis was carried out by computing overlaps with canonical pathways and gene ontology gene sets obtained from the Broad Institute.

**Results and Discussion**

The efficacy of DM Score to predict MMCs sensitivity to 5-azacitidine was investigated on 12 HMCLs with high or low DM Score. The 6 HMCLs with high DM Score exhibited a 3 fold higher 5-azacitidine sensitivity (P = 0.01; median IC50 = 2.43 $\mu$M; range: 1.12 to 8.25 $\mu$M) compared to the 6 ones with a low DM Score (median IC50 = 7.45 $\mu$M; range: 5.73 to 27.9) (Figure 5). DM score could also predict for the ability of 5-azacytidine to kill patients' primary MMCs cultured together with their BM environment. The MMCs of patients with a high DM Score (N = 7) exhibited a significant 1.6 fold higher 5-azacytidine sensitivity compared to low DM Score patients (N = 7) (P < .05, Figure 6).

Thus, the DM Score, which was built using 47 genes whose expression is deregulated by decitabine in HMCLs and which have prognostic value for patients overall survival, can predict for the sensitivity of MM cell lines and primary MMCs to the two-clinical grade inhibitors of DNMT. These 47 genes include 22 genes associated with a bad prognosis and 25 with good one. Using GSEA analysis, MMCs of patients with a high DM Score show a significant enrichment in genes associated with proliferation (gene sets: REACTOME CELL CYCLE, CELL CYCLE G2 M and PLASMA CELLS VS PLASMABLAST DN, P<0.001, and supplementary Tables S7, S8 and S9). On the other hand, MMCs of patients with a low DM Score show a significant enrichment in genes coding for solute carrier group of membrane transport proteins (gene sets: REACTOME TRANSPORT OF INORGANIC CATIONS ANIONS AND AMINO ACIDS OLIGOPEPTIDES, P<0.001, and supplementary Tables S10).

Thus, the higher sensitivity of MMCs of patients with a high DM Score to DNMT inhibitors could be explained by the fact that these inhibitors are mainly active in cell cycling cells since incorporation into DNA is restricted to the S-phase (Hollenbach, et al 2010), and also by a reduced drug export in MMCs of these patients, resulting in higher intracellular drug accumulation.

Clinical trials evaluate the safety of DNMTi as monotherapy or in combination with lenalidomide or dexamethasone in MM (Maes, et al 2013; Toor, et al 2012) and investigate the link between HM Score and response of patients to DNMTi could be promising.

In conclusion, the DM Score allows identification of MM patients who could benefit from treatment with two clinical grade DNMT inhibitors and the development of personalized treatment.

**TABLE S7: Genes set enrichment analysis revealed a significant overrepresentation of the REACTOME CELL CYCLEset in high DM Score patients compared to low DM Score patients (*P*<.001).**

| PROBE | GENE SYMBOL | GENE_TITLE | RANK METRIC SCORE | RUNNING Enrichment Score | CORE ENRIC HMENT |
|---|---|---|---|---|---|
| MYBL2 | MYBL2 | v-myb myeloblastosis viral oncogene homolog (avian)-like 2 | -0.167122021317482 | -0.6420408 | Yes |
| CDKN2C | CDKN2C | cyclin-dependent kinase inhibitor 2C (p18, inhibits CDK4) | -0.16787339746952057 | -0.626822 | Yes |
| CDK6 | CDK6 | cyclin-dependent kinase 6 | -0.19776728749275208 | -0.6317126 | Yes |
| LMNA | LMNA | lamin A/C | -0.20474471151828766 | -0.61800563 | Yes |
| CDKN2B | CDKN2B | cyclin-dependent kinase inhibitor 2B (p15, inhibits CDK4) | -0.22542211413383484 | -0.6069075 | Yes |
| TYMS | TYMS | thymidylate synthetase | -0.22692811489105225 | -0.5853221 | Yes |
| MCM10 | MCM10 | MCM10 minichromosome maintenance deficient 10 (S. cerevisiae) | -0.23174770176410675 | -0.56442255 | Yes |
| BUB1B | BUB1B | BUB1 budding uninhibited by benzimidazoles 1 homolog beta (yeast) | -0.2420835942029953 | -0.54413825 | Yes |
| KIF23 | KIF23 | kinesin family member 23 | -0.25110968947410583 | -0.52252656 | Yes |
| CHEK1 | CHEK1 | CHK1 checkpoint homolog (S. pombe) | -0.2538430988788605 | -0.49904326 | Yes |
| HIST1H4H | HIST1H4H | histone cluster 1, H4h | -0.27717286348342896 | -0.47906303 | Yes |
| MCM2 | MCM2 | MCM2 minichromosome maintenance deficient 2, mitotin (S. cerevisiae) | -0.29232123494148254 | -0.45348957 | Yes |
| GINS1 | GINS1 | GINS complex subunit 1 (Psf1 homolog) | -0.29381972551345825 | -0.42524388 | Yes |
| KIF20A | KIF20A | kinesin family member 20A | -0.30122432112693787 | -0.3985847 | Yes |
| MCM4 | MCM4 | MCM4 minichromosome maintenance deficient 4 (S. cerevisiae) | -0.3015007972717285 | -0.36983046 | Yes |
| OIP5 | OIP5 | Opa interacting protein 5 | -0.30586564540863037 | -0.3406566 | Yes |
| CCND2 | CCND2 | cyclin D2 | -0.31117963790893555 | -0.31189123 | Yes |
| RRM2 | RRM2 | ribonucleotide reductase M2 polypeptide | -0.3157253563404083 | -0.28176954 | Yes |
| BIRC5 | BIRC5 | baculoviral IAP repeat-containing 5 (survivin) | -0.3474469482898712 | -0.2515862 | Yes |
| AURKA | AURKA | aurora kinase A | -0.35163724422454834 | -0.21778236 | Yes |
| CENPA | CENPA | centromere protein A | -0.35916951298713684 | -0.1837141 | Yes |
| CCNB2 | CCNB2 | cyclin B2 | -0.36041226983070374 | -0.1490667 | Yes |

(continued)

| PROBE | GENE SYMBOL | GENE_TITLE | RANK METRIC SCORE | RUNNING Enrichment Score | CORE ENRICHMENT |
|---|---|---|---|---|---|
| HIST1H4 | HIST1H4C | histone cluster 1, H4c | -0.37045058608055115 | -0.11368413 | Yes |
| CCNB1 | CCNB1 | cyclin B 1 | -0.38353657722473145 | -0.077273406 | Yes |
| CDCA8 | CDCA8 | cell division cycle associated 8 | -0.39406412839889526 | -0.039620794 | Yes |
| NEK2 | NEK2 | NIMA (never in mitosis gene a)-related kinase 2 | -0.4169292151927948 | 2.2989404E-4 | Yes |

**Supplementary Table S8: Genes set enrichment analysis revealed a significant overrepresentation of the CELL CYCLE G2/M set in high DM Score patients compared to low DM Score patients (*P*<.001).**

| PROBE | GENE SYMBOL | GENE_TITLE | RANK METRIC SCORE | RUNNING Enrichment Score | CORE ENRICHMENT |
|---|---|---|---|---|---|
| CENPE | CENPE | centromere protein E, 312kDa | -0.15664561092853546 | -0.6155994 | Yes |
| BMP2 | BMP2 | bone morphogenetic protein 2 | -0.16032224893569946 | -0.6001657 | Yes |
| TACC3 | TACC3 | transforming, acidic coiled-coil containing protein 3 | -0.18774476647377014 | -0.6039366 | Yes |
| PRR5 | PRR5 | proline rich 5 (renal) | -0.18954475224018097 | -0.582728 | Yes |
| CENPF | CENPF | centromere protein F, 350/400ka (mitosin) | -0.19975358247756958 | -0.5653478 | Yes |
| LMNA | LMNA | lamin A/C | -0.20474471151828766 | -0.5455384 | Yes |
| TPX2 | TPX2 | TPX2, microtubule-associated, homolog (Xenopus laevis) | -0.224465506196022034 | -0.52863073 | Yes |
| SHCBP1 | SHCBP1 | SHC SH2-domain binding protein 1 | -0.23987287282943726 | -0.5050944 | Yes |
| KIF14 | KIF14 | kinesin family member 14 | -0.2565326690673828 | -0.48026097 | Yes |
| DEPDC1 B | DEPDC1B | DEP domain containing 1B | -0.2718331515789032 | -0.4513114 | Yes |
| HMMR | HMMR | hyaluronan-mediated motility receptor (RHAMM) | -0.2835068106651306 | -0.42005372 | Yes |
| DEPDC1 | DEPDC1 | DEP domain containing 1 | -0.28364425897598267 | -0.3862573 | Yes |
| ANLN | ANLN | anillin, actin binding protein | -0.286072313785553 | -0.35217157 | Yes |
| HMGB3 | HMGB3 | high-mobility group box 3 | -0.28622567653656006 | -0.31806755 | Yes |
| MCM4 | MCM4 | MCM4 minichromosome maintenance deficient 4 (S. cerevisiae) | -0.3015007972717285 | -0.286271 | Yes |
| FOXM1 | FOXM1 | forkhead box M1 | -0.3144078850746155 | -0.25064352 | Yes |
| CEP55 | CEP55 | centrosomal protein 55kDa | -0.3161003887653351 | -0.21320921 | Yes |
| BIRC5 | BIRC5 | baculoviral IAP repeat-containing 5 (survivin) | -0.3474469482898712 | -0.17479162 | Yes |

(continued)

| PROBE | GENE SYMBOL | GENE_TITLE | RANK METRIC SCORE | RUNNING Enrichment Score | CORE ENRICHMENT |
|-------|-------------|------------|-------------------|--------------------------|-----------------|
| AURKA | AURKA | aurora kinase A | -0.35163724422454834 | -0.13289377 | Yes |
| CENPA | CENPA | centromere protein A | -0.35916951298713684 | -0.09055706 | Yes |
| CCNB2 | CCNB2 | cyclin B2 | -0.36041226983070374 | -0.047613665 | Yes |
| NEK2 | NEK2 | NIMA (never in mitosis gene a)-related kinase 2 | -0.4169292151927948 | 2.2932523E-4 | Yes |

| PROBE | GENE SYMBOL | GENE_TITLE | RANK METRIC SCORE | RUNNING Enrichment Score | CORE ENRICHMENT |
|-------|-------------|------------|-------------------|--------------------------|-----------------|

**Supplementary Table S9: Genes set enrichment analysis revealed a significant overrepresentation of the PLASMA CELLS VS PLASMABLAST set in high DM Score patients compared to low DM Score patients (*P*<.001).**

| PROBE | GENE SYMBOL | GENE_TITLE | RANK METRIC SCORE | RUNNING Enrichment Score | CORE ENRICHMENT |
|---|---|---|---|---|---|
| CLEC2B | CLEC2B | C-type lectin domain family 2, member B | -0.1298338621854782 | -0.6410949 | Yes |
| PLK4 | PLK4 | polo-like kinase 4 (Drosophila) | -0.130959272238464355 | -0.62984425 | Yes |
| RNASE6 | RNASE6 | ribonuclease, RNase A family, k6 | -0.13925980031490326 | -0.62673587 | Yes |
| IFI30 | IFI30 | interferon, gamma-inducible protein 30 | -0.1412345916032791 | -0.61493564 | Yes |
| GLIPR1 | GLIPR1 | GLI pathogenesis-related 1 (glioma) | -0.141678586602211 | -0.6014841 | Yes |
| CENPE | CENPE | centromere protein E, 312kDa | -0.15664561092853546 | -0.60218084 | Yes |
| CD52 | CD52 | CD52 molecule | -0.15694235265254974 | -0.58677125 | Yes |
| ITGB1 | ITGB1 | integrin, beta 1 (fibronectin receptor, beta polypeptide, antigen CD29 includes MDF2, MSK12) | -0.1667313575744629 | -0.5811952 | Yes |
| PAPOL A | PAPOLA | poly(A) polymerase alpha | -0.19660720229148865 | -0.5871552 | Yes |
| GALNT 3 | GALNT3 | UDP-N-acetyl-alpha-D-galactosamine:polypeptide N-acetylgalactosaminyltransferase 3 (GalNAc-T3) | -0.19789130985736847 | -0.56841403 | Yes |
| CD58 | CD58 | CD58 molecule | -0.21272079646587372 | -0.5571741 | Yes |
| GLDC | GLDC | glycine dehydrogenase (decarboxylating) | -0.22228969633579254 | -0.5404011 | Yes |
| TYMS | TYMS | thymidylate synthetase | -0.22692811489105225 | -0.5197276 | Yes |
| GZMB | GZMB | granzyme B (granzyme 2, cytotoxic T-lymphocyte-associated serine esterase 1) | -0.2282218039035797 | -0.49754903 | Yes |
| ITGB7 | ITGB7 | integrin, beta 7 | -0.24833402037620544 | -0.47867823 | Yes |
| KIF23 | KIF23 | kinesin family member 23 | -0.25110968947410583 | -0.45517108 | Yes |
| KIF14 | KIF14 | kinesin family member 14 | -0.2565326690673828 | -0.43227983 | Yes |
| PFKP | PFKP | phosphofructokinase, platelet | -0.2628379166126251 | -0.40693212 | Yes |
| HMMR | HMMR | hyaluronan-mediated motility receptor (RHAMM) | -0.2835068106651306 | -0.38460782 | Yes |
| GINS1 | GINS1 | GINS complex subunit 1 (Psfl homolog) | -0.29381972551345825 | -0.35759616 | Yes |
| SELL | SELL | selectin L (lymphocyte adhesion molecule 1) | -0.2999555468559265 | -0.32998204 | Yes |

EP 2 898 091 B1

(continued)

| PROBE | GENE SYMBOL | GENE_TITLE | RANK METRIC SCORE | RUNNING Enrichment Score | CORE ENRICH MENT |
|---|---|---|---|---|---|
| TOP2A | TOP2A | topoisomerase (DNA) II alpha 170kDa | -0.30873504281044006 | -0.3015059 | Yes |
| CDKN3 | CDKN3 | cyclin-dependent kinase inhibitor 3 (CDK2-associated dual specificity phosphatase) | -0.30915674567222595 | -0.27138063 | Yes |
| CCND2 | CCND2 | cyclin D2 | -0.31117963790893555 | -0.24082708 | Yes |
| FOXM1 | FOXM1 | forkhead box M1 | -0.3144078850746155 | -0.20995656 | Yes |
| RRM2 | RRM2 | ribonucleotide reductase M2 polypeptide | -0.3157253563404083 | -0.17895667 | Yes |
| CASP6 | CASP6 | caspase 6, apoptosis-related cysteine peptidase | -0.34289273619651794 | -0.14781573 | Yes |
| CENPA | CENPA | centromere protein A | -0.35916951298713684 | -0.11392827 | Yes |
| S100A10 | S100A10 | S100 calcium binding protein A10 | -0.37857523560523987 | -0.07767609 5 | Yes |
| CCNB1 | CCNB1 | cyclin B1 | -0.38353657722473145 | -0.040018078 | Yes |
| NEK2 | NEK2 | NIMA (never in mitosis gene a)-related kinase 2 | -0.4169292151927948 | 2.296113E-4 | Yes |

**Supplementary Table S10: Genes set enrichment analysis revealed a significant overrepresentation of the REACTOME TRANSPORT OF INORGANIC CATIONS ANIONS AND AMINO ACID OLIGOPEPTIDES set in low DM Score patients compared to high DM Score patients (*P*<.001).**

| PROBE | GENE SYMBOL | GENE_TITLE | RANK METRIC SCORE | RUNNING Enrichment Score | CORE ENRICH MENT |
|---|---|---|---|---|---|
| SLC24A3 | SLC24A3 | solute carrier family 24 (sodium/potassium/calcium exchanger), member 3 | 0.18617968261241913 | 0.03475809 | Yes |
| SLC17A8 | SLC17A8 | solute carrier family 17 (sodium-dependent inorganic phosphate cotransporter), member 8 | 0.17720697820186615 | 0.09601015 | Yes |
| SLC7A8 | SLC7A8 | solute carrier family 7 (cationic amino acid transporter, y+ system), member 8 | 0.17013469338417053 | 0.15928961 | Yes |
| SLC1A1 | SLC1A1 | solute carrier family 1 (neuronal/epithelial high affinity glutamate transporter, system Xag), member 1 | 0.15042848885059357 | 0.19263873 | Yes |
| SLC4A1 | SLC4A1 | solute carrier family 4, anion exchanger, member 1 (erythrocyte membrane protein band 3, Diego blood group) | 0.134476438164711 | 0.21448465 | Yes |
| SLC12A5 | SLC12A5 | solute carrier family 12, (potassium-chloride transporter) member 5 | 0.13315615057945251 | 0.26718774 | Yes |
| SLC9A3 | SLC9A3 | solute carrier family 9 (sodium/ hydrogen exchanger), member 3 | 0.1316232681274414 | 0.3169685 | Yes |
| SLC15A2 | SLC15A2 | solute carrier family 15 (H+/ peptide transporter), member 2 | 0.11438794434070587 | 0.3207876 | Yes |
| SLC4A4 | SLC4A4 | solute carrier family 4, sodium bicarbonate cotransporter, member 4 | 0.10706888139247894 | 0.34565148 | Yes |
| SLC12A1 | SLC12A1 | solute carrier family 12 (sodium/potassium/chloride transporters), member 1 | 0.106844961643219 | 0.3904528 | Yes |
| SLC24A5 | SLC24A5 | solute carrier family 24, member 5 | 0.09523425251245499 | 0.39576042 | Yes |
| SLC8A1 | SLC8A1 | solute carrier family 8 (sodium/ calcium exchanger), member 1 | 0.09496445208787918 | 0.43509954 | Yes |
| SLC1A3 | SLC1A3 | solute carrier family 1 (glial high affinity glutamate transporter), member 3 | 0.09355431795120239 | 0.47225094 | Yes |

(continued)

| PROBE | GENE SYMBOL | GENE_TITLE | RANK METRIC SCORE | RUNNING Enrichment Score | CORE ENRICH MENT |
|---|---|---|---|---|---|
| SLC8A3 | SLC8A3 | solute carrier family 8 (sodium-calcium exchanger), member 3 | 0.08295272290706635 | 0.47784585 | Yes |

**REFERENCES:**

Throughout this application, various references describe the state of the art to which this invention pertains.

1. Hahn WC, Weinberg RA. Rules for making human tumor cells. N Engl J Med. 2002;347(20):1593-1603.

2. Vogelstein B, Kinzler KW. Cancer genes and the pathways they control. Nat Med. 2004;10(8):789-799.

3. Baylin SB. DNA methylation and gene silencing in cancer. Nat Clin Pract Oncol. 2005;2 Suppl 1:S4-11.

4. Kondo Y. Epigenetic cross-talk between DNA methylation and histone modifications in human cancers. Yonsei Med J. 2009;50(4):455-463.

5. Issa JP. DNA methylation as a therapeutic target in cancer. Clin Cancer Res. 2007;13(6):1634-1637.

6. Issa JP, Garcia-Manero G, Giles FJ, et al. Phase 1 study of low-dose prolonged exposure schedules of the hypomethylating agent 5-aza-2'-deoxycytidine (decitabine) in hematopoietic malignancies. Blood. 2004;103(5):1635-1640.

7. Oki Y, Jelinek J, Shen L, Kantarjian HM, Issa JP. Induction of hypomethylation and molecular response after decitabine therapy in patients with chronic myelomonocytic leukemia. Blood. 2008;111(4):2382-2384.

8. Smith EM, Boyd K, Davies FE. The potential role of epigenetic therapy in multiple myeloma. Br J Haematol. 2009.

9. Bergsagel PL, Kuehl WM. Molecular pathogenesis and a consequent classification of multiple myeloma. J Clin Oncol. 2005;23(26):6333-6338.

10. Hideshima T, Bergsagel PL, Kuehl WM, Anderson KC. Advances in biology of multiple myeloma: clinical applications. Blood. 2004;104(3):607-618.

11. Chen G, Wang Y, Huang H, et al. Combination of DNA methylation inhibitor 5-azacytidine and arsenic trioxide has synergistic activity in myeloma. Eur J Haematol. 2009;82(3):176-183.

12. de Carvalho F, Colleoni GW, Almeida MS, Carvalho AL, Vettore AL. TGFbetaR2 aberrant methylation is a potential prognostic marker and therapeutic target in multiple myeloma. Int J Cancer. 2009;125(8):1985-1991.

13. Galm O, Yoshikawa H, Esteller M, Osieka R, Herman JG. SOCS-1, a negative regulator of cytokine signaling, is frequently silenced by methylation in multiple myeloma. Blood. 2003;101(7):2784-2788.

14. Hatzimichael E, Dranitsaris G, Dasoula A, et al. Von Hippel-Lindau methylation status in patients with multiple myeloma: a potential predictive factor for the development of bone disease. Clin Lymphoma Myeloma. 2009;9(3):239-242.

15. Hodge DR, Peng B, Cherry JC, et al. Interleukin 6 supports the maintenance of p53 tumor suppressor gene promoter methylation. Cancer Res. 2005;65(11):4673-4682.

16. Ng MH, Chung YF, Lo KW, Wickham NW, Lee JC, Huang DP. Frequent hypermethylation of p16 and p15 genes in multiple myeloma. Blood. 1997;89(7):2500-2506.

17. Seidl S, Ackermann J, Kaufmann H, et al. DNA-methylation analysis identifies the E-cadherin gene as a potential marker of disease progression in patients with monoclonal gammopathies. Cancer. 2004;100(12):2598-2606.

18. Tshuikina M, Jernberg-Wiklund H, Nilsson K, Oberg F. Epigenetic silencing of the interferon regulatory factor ICSBP/IRF8 in human multiple myeloma. Exp Hematol. 2008;36(12):1673-1681.

19. Heller G, Schmidt WM, Ziegler B, et al. Genome-wide transcriptional response to 5-aza-2'-deoxycytidine and trichostatin a in multiple myeloma cells. Cancer Res. 2008;68(1):44-54.

20. Wilop S, van Gemmeren TB, Lentjes MH, et al. Methylation-associated dysregulation of the suppressor of cytokine signaling-3 gene in multiple myeloma. Epigenetics. 2011;6(8):1047-1052.

21. Walker BA, Wardell CP, Chiecchio L, et al. Aberrant global methylation patterns affect the molecular pathogenesis and prognosis of multiple myeloma. Blood. 2010.

22. Moreaux J, Klein B, Bataille R, et al. A high-risk signature for patients with multiple myeloma established from the molecular classification of human myeloma cell lines. Haematologica. 2011;96(4):574-582.

23. Zhang XG, Gaillard JP, Robillard N, et al. Reproducible obtaining of human myeloma cell lines as a model for tumor stem cell study in human multiple myeloma. Blood. 1994;83(12):3654-3663.

24. Rebouissou C, Wijdenes J, Autissier P, et al. A gp130 interleukin-6 transducer-dependent SCID model of human multiple myeloma. Blood. 1998;91(12):4727-4737.

25. Tarte K, Zhang XG, Legouffe E, et al. Induced expression of B7-1 on myeloma cells following retroviral gene transfer results in tumor-specific recognition by cytotoxic T cells. J Immunol. 1999;163(1):514-524.

26. Gu ZJ, Vos JD, Rebouissou C, et al. Agonist anti-gp130 transducer monoclonal antibodies are human myeloma cell survival and growth factors. Leukemia. 2000;14(1):188-197.

27. Goldschmidt H, Sonneveld P, Cremer FW, et al. Joint HOVON-50/GMMG-HD3 randomized trial on the effect of thalidomide as part of a high-dose therapy regimen and as maintenance treatment for newly diagnosed myeloma patients. Ann Hematol. 2003;82(10):654-659.

28. Cremer FW, Bila J, Buck I, et al. Delineation of distinct subgroups of multiple myeloma and a model for clonal evolution based on interphase cytogenetics. Genes Chromosomes Cancer. 2005;44(2):194-203.

29. Barlogie B, Tricot G, Rasmussen E, et al. Total therapy 2 without thalidomide in comparison with total therapy 1: role of intensified induction and posttransplantation consolidation therapies. Blood. 2006;107(7):2633-2638.

30. Sprynski AC, Hose D, Caillot L, et al. The role of IGF-1 as a major growth factor for myeloma cell lines and the prognostic relevance of the expression of its receptor. Blood. 2009;113(19):4614-4626.

31. Xiong W, Wu X, Starnes S, et al. An analysis of the clinical and biologic significance of TP53 loss and the identification of potential novel transcriptional targets of TP53 in multiple myeloma. Blood. 2008;112(10):4235-4246.

32. Moreaux J, Legouffe E, Jourdan E, et al. BAFF and APRIL protect myeloma cells from apoptosis induced by interleukin 6 deprivation and dexamethasone. Blood. 2004;103(8):3148-3157.

33. Hose D, Reme T, Meissner T, et al. Inhibition of aurora kinases for tailored risk-adapted treatment of multiple myeloma. Blood. 2009;113(18):4331-4340.

34. Moreaux J, Cremer FW, Reme T, et al. The level of TACI gene expression in myeloma cells is associated with a signature of microenvironment dependence versus a plasmablastic signature. Blood. 2005;106(3):1021-1030.

35. Reme T, Hose D, De Vos J, et al. A new method for class prediction based on signed-rank algorithms applied to Affymetrix microarray experiments. BMC Bioinformatics. 2008;9:16.

36. Tanguy Le Carrour SA, Sylvie Tondeur, Ludovic Lhermitte, Ned Lamb, Thierry Reme, Veronique Pantesco, Samir Hamamah, Bernard Klein, John De Vos. Amazonia!: An Online Resource to Google and Visualize Public Human whole Genome Expression Data. The Open Bioinformatics Journal. 2010;4:5-10.

37. Cui X, Churchill GA. Statistical tests for differential expression in cDNA microarray experiments. Genome Biol. 2003;4(4):210.

38. Kassambara A, Hose D, Moreaux J, et al. Genes with a spike expression are clustered in chromosome (sub)bands and spike (sub)bands have a powerful prognostic value in patients with multiple myeloma. Haematologica. 2011.

39. Zhan F, Huang Y, Colla S, et al. The molecular classification of multiple myeloma. Blood. 2006;108(6):2020-2028.

40. Chu WK, Hickson ID. RecQ helicases: multifunctional genome caretakers. Nat Rev Cancer. 2009;9(9):644-654.

41. Harrigan JA, Bohr VA. Human diseases deficient in RecQ helicases. Biochimie. 2003;85(11):1185-1193.

42. Hickson ID. RecQ helicases: caretakers of the genome. Nat Rev Cancer. 2003;3(3):169-178.

43. Arai A, Chano T, Futami K, et al. RECQL1 and WRN proteins are potential therapeutic targets in head and neck squamous cell carcinoma. Cancer Res. 2011;71(13):4598-4607.

44. Futami K, Kumagai E, Makino H, et al. Induction of mitotic cell death in cancer cells by small interference RNA suppressing the expression of RecQL1 helicase. Cancer Sci. 2008;99(1):71-80.

45. Futami K, Kumagai E, Makino H, et al. Anticancer activity of RecQL1 helicase siRNA in mouse xenograft models. Cancer Sci. 2008;99(6):1227-1236.

46. Mendoza-Maldonado R, Faoro V, Bajpai S, et al. The human RECQ1 helicase is highly expressed in glioblastoma and plays an important role in tumor cell proliferation. Mol Cancer. 2011;10:83.

47. Miki H, Okada Y, Hirokawa N. Analysis of the kinesin superfamily: insights into structure and function. Trends Cell Biol. 2005;15(9):467-476.

48. Hirokawa N. Kinesin and dynein superfamily proteins and the mechanism of organelle transport. Science. 1998;279(5350):519-526.

49. Yu Y, Feng YM. The role of kinesin family proteins in tumorigenesis and progression: potential biomarkers and molecular targets for cancer therapy. Cancer. 2010;116(22):5150-5160.

50. Zhu C, Zhao J, Bibikova M, et al. Functional analysis of human microtubule-based motor proteins, the kinesins and dyneins, in mitosis/cytokinesis using RNA interference. Mol Biol Cell. 2005;16(7):3187-3199.

51. Avet-Loiseau H, Li C, Magrangeas F, et al. Prognostic significance of copy-number alterations in multiple myeloma. J Clin Oncol. 2009;27(27):4585-4590.

52. Agnelli L, Forcato M, Ferrari F, et al. The reconstruction of transcriptional networks reveals critical genes with implications for clinical outcome of multiple myeloma. Clin Cancer Res. 2011;17(23):7402-7412.

53. De Vos, J., Thykjaer, T., Tarte, K., Ensslen, M., Raynaud, P., Requirand, G., Pellet, F., Pantesco, V., Reme, T., Jourdan, M., Rossi, J.F., Orntoft, T. & Klein, B. (2002) Comparison of gene expression profiling between malignant and normal plasma cells with oligonucleotide arrays. Oncogene, 21, 6848-6857.

54. Flotho, C., Claus, R., Batz, C., Schneider, M., Sandrock, I., Ihde, S., Plass, C., Niemeyer, C.M. & Lubbert, M. (2009) The DNA methyltransferase inhibitors azacitidine, decitabine and zebularine exert differential effects on cancer gene expression in acute myeloid leukemia cells. Leukemia, 23, 1019-1028.

55. Ghoshal, K., Datta, J., Majumder, S., Bai, S., Kutay, H., Motiwala, T. & Jacob, S.T. (2005) 5-Aza-deoxycytidine induces selective degradation of DNA methyltransferase 1 by a proteasomal pathway that requires the KEN box, bromo-adjacent homology domain, and nuclear localization signal. Mol Cell Biol, 25, 4727-4741.

56. Heuck, C.J., Mehta, J., Bhagat, T., Gundabolu, K., Yu, Y., Khan, S., Chrysofakis, G., Schinke, C., Tariman, J., Vickrey, E., Pulliam, N., Nischal, S., Zhou, L., Bhattacharyya, S., Meagher, R., Hu, C., Maqbool, S., Suzuki, M., Parekh, S., Reu, F., Steidl, U., Greally, J., Verma, A. & Singhal, S.B. (2013) Myeloma is characterized by stage-specific alterations in DNA methylation that occur early during myelomagenesis. J Immunol, 190, 2966-2975.

57. Hollenbach, P.W., Nguyen, A.N., Brady, H., Williams, M., Ning, Y., Richard, N., Krushel, L., Aukerman, S.L., Heise, C. & MacBeth, K.J. (2010) A comparison of azacitidine and decitabine activities in acute myeloid leukemia cell lines. PLoS One, 5, e9001.

58. Jourdan, M., Ferlin, M., Legouffe, E., Horvathova, M., Liautard, J., Rossi, J.F., Wijdenes, J., Brochier, J. & Klein, B. (1998) The myeloma cell antigen syndecan-1 is lost by apoptotic myeloma cells. Br J Haematol, 100, 637-646.

59. Kaiser, M.F., Johnson, D.C., Wu, P., Walker, B.A., Brioli, A., Mirabella, F., Wardell, C.P., Melchor, L., Davies, F.E. & Morgan, G.J. (2013) Global methylation analysis identifies prognostically important epigenetically inactivated tumour suppressor genes in multiple myeloma. Blood.

60. Kiziltepe, T., Hideshima, T., Catley, L., Raje, N., Yasui, H., Shiraishi, N., Okawa, Y., Ikeda, H., Vallet, S., Pozzi, S., Ishitsuka, K., Ocio, E.M., Chauhan, D. & Anderson, K.C. (2007) 5-Azacytidine, a DNA methyltransferase inhibitor, induces ATR-mediated DNA double-strand break responses, apoptosis, and synergistic cytotoxicity with doxorubicin and bortezomib against multiple myeloma cells. Mol Cancer Ther, 6, 1718-1727.

61. Maes, K., Menu, E., Van Valckenborgh, E., Van Riet, I., Vanderkerken, K. & De Bruyne, E. (2013) Epigenetic Modulating Agents as a New Therapeutic Approach in Multiple Myeloma. Cancers, 5, 430-461.

62. Mahtouk, K., Jourdan, M., De Vos, J., Hertogh, C., Fiol, G., Jourdan, E., Rossi, J.F. & Klein, B. (2004) An inhibitor of the EGF receptor family blocks myeloma cell growth factor activity of HB-EGF and potentiates dexamethasone or anti-IL-6 antibody-induced apoptosis. Blood, 103, 1829-1837.

63. Moreaux, J., Reme, T., Leonard, W., Veyrune, J.L., Requirand, G., Goldschmidt, H., Hose, D. & Klein, B. (2012) Development of gene expression-based score to predict sensitivity of multiple myeloma cells to DNA methylation inhibitors. Mol Cancer Ther, 11, 2685-2692.

64. Palii, S.S., Van Emburgh, B.O., Sankpal, U.T., Brown, K.D. & Robertson, K.D. (2008) DNA methylation inhibitor 5-Aza-2'-deoxycytidine induces reversible genome-wide DNA damage that is distinctly influenced by DNA methyltransferases 1 and 3B. Mol Cell Biol, 28, 752-771.

65. Rodriguez-Paredes, M. & Esteller, M. (2011) Cancer epigenetics reaches mainstream oncology. Nat Med, 17, 330-339.

66. Stresemann, C., Brueckner, B., Musch, T., Stopper, H. & Lyko, F. (2006) Functional diversity of DNA methyltransferase inhibitors in human cancer cell lines. Cancer Res, 66, 2794-2800.

67. Tanguy Le Carrour, S.A., Sylvie Tondeur, Ludovic Lhermitte, Ned Lamb, Thierry Reme, Veronique Pantesco, Samir Hamamah, Bernard Klein, John De Vos (2010) Amazonia!: An Online Resource to Google and Visualize Public Human whole Genome Expression Data. The Open Bioinformatics Journal, 4, 5-10.

68. Toor, A.A., Payne, K.K., Chung, H.M., Sabo, R.T., Hazlett, A.F., Kmieciak, M., Sanford, K., Williams, D.C., Clark, W.B., Roberts, C.H., McCarty, J.M. & Manjili, M.H. (2012) Epigenetic induction of adaptive immune response in multiple myeloma: sequential azacitidine and lenalidomide generate cancer testis antigen-specific cellular immunity. Br J Haematol, 158, 700-711.

69. Walker, B.A., Wardell, C.P., Chiecchio, L., Smith, E.M., Boyd, K.D., Neri, A., Davies, F.E., Ross, F.M. & Morgan, G.J. (2010) Aberrant global methylation patterns affect the molecular pathogenesis and prognosis of multiple myeloma. Blood.

70. Zhang, X.G., Gaillard, J.P., Robillard, N., Lu, Z.Y., Gu, Z.J., Jourdan, M., Boiron, J.M., Bataille, R. & Klein, B. (1994) Reproducible obtaining of human myeloma cell lines as a model for tumor stem cell study in human multiple myeloma. Blood, 83, 3654-3663.

71. Vanderkerken, K. et al. (2011) Epigenetic changes of myeloma cells within the bone marrow microenvironment. Haematologica, Abstract Book 13th International Myeloma Workshop, Paris, France, May 3-6, 2011,vol. 96, no. supplement 11 May 2011.

72. Smith, E.M. et al. (2010) The potential role of epigenetic therapy in multiple myeloma. Br J Haematol, vol. 148, no. 5, 1 March 2010 pages 702-713.

**Claims**

1. A method of testing whether a patient suffering of multiple myeloma will respond or not to a DNA methyltransferase inhibitor (DNMTi) comprising:

 i. determining the expression level (ELi) of the 47 genes $G_1$-$G_{47}$ from table A in a biological sample obtained from said patient
 ii. comparing the expression level (ELi) determined at step i) with a predetermined reference level (ELRi)
 iii. calculating the DMS score trough the following formula

$$DMS = \sum_{i=1}^{n} \beta i \times Ci$$

 wherein $\beta i$ represent the regression $\beta$ coefficient reference value for the gene $G_i$ and $Ci$ = 1 if the expression of the gene $G_i$ (ELi) is higher than the predetermined reference level (ELRi) or $Ci$ = -1 if the expression of the gene (ELi) is lower than or equal to the predetermined reference level (ELRi)
 iv. comparing the score DMS determined at step iii) with a predetermined reference value $DMS_R$
 v. and concluding that the patient will respond to the DNMTi when the DMS score is higher than the predetermined reference value $DMS_R$ or concluding that the patient will not respond to the DNMTi when the DMS score is lower than the predetermined reference value $DMS_R$.

2. The method according to claim 1, wherein the said DNA methyltransferase inhibitor is a nucleoside analogue.

3. The method according to claim 2, wherein the nucleoside analogue is selected in the group consisting of 5-Azacytidine (azacytidine), 5-Aza-2'-deoxycytidine (decitabine, 5-Aza-CdR), zebularine, 5-Fluoro-2'-deoxycytidine (5-F-CdR), 5,6-Dihydro-5-azacytidine (DHAC).

4. The method according to claim 2, wherein the said DNA methyltransferase inhibitor is a non-nucleoside analogue.

5. The method according to claim 4, wherein the non-nucleoside analogue is selected in the group consisting of Hydralazine, Procainamide, Procaine, EGCG ((-)-epigallocatechin-3-gallate), Psammaplin A, MG98, RG108.

6. A DNA methyltransferase inhibitor for its use in the treatment of multiple myeloma in a patient in need thereof, wherein the patient was tested with the method according to claim 1 wherein the predetermined reference value $DMS_R$ is the median value of DMS, and wherein the said patient is classified as responder by the method according to claim 1.

7. The DNA methyltransferase inhibitor for its use according to claim 6, wherein the said DNA methyltransferase inhibitor is a nucleoside analogue.

8. The DNA methyltransferase inhibitor for its use according to claim 7, wherein the nucleoside analogues are selected in the group consisting of 5-Azacytidine (azacytidine), 5-Aza-2'-deoxycytidine (decitabine, 5-Aza-CdR), zebularine, 5-Fluoro-2'-deoxycytidine (5-F-CdR), 5,6-Dihydro-5-azacytidine (DHAC).

9. The DNA methyltransferase inhibitor for its use according to claim 6, wherein the said DNA methyltransferase inhibitor is a non-nucleoside analogue.

10. The DNA methyltransferase inhibitor for its use according to claim 9, wherein the non-nucleoside analogues are selected in the group consisting of Hydralazine, Procainamide, Procaine, EGCG ((-)-epigallocatechin-3-gallate), Psammaplin A, MG98, RG108.

**Patentansprüche**

1. Verfahren zum Testen ob ein Patient, der an multiplem Myelom leidet, auf einen DNA-Methyltransferase-Inhibitor (DNMTi) antworten wird oder nicht, umfassend:

i. Bestimmen des Expressionslevels (ELi) von den 47 Genen $G_1$-$G_{47}$ aus Table A in einer biologischen, von dem Patienten erhaltenen Probe

ii. Vergleichen des in Schritt i) bestimmten Expressionslevels (ELi) mit einem vorbestimmten Referenzlevel (ELRi)

iii. Berechnen des DMS-Scores anhand der folgenden Formel

$$DMS = \sum_{i=1}^{n} \beta i \times Ci$$

worin $\beta i$ für den Regressions-$\beta$-Koeffizient-Referenzwert für das Gen $G_i$ steht und $Ci$ = 1 ist, wenn die Expression des Gens $G_i$ (ELi) höher ist als der vorbestimmte Referenzlevel (ELRi) oder $Ci$ = -1 ist, wenn die Expression des Gens (ELi) niedriger als oder gleich dem vorbestimmten Referenzlevel (ELRi) ist

iv. Vergleichen des in Schritt iii) bestimmten Scores DMS mit einem vorbestimmten Referenzwert $DMS_R$

v. und Folgern, dass der Patient auf den DNMTi antworten wird, wenn der DMS-Score höher ist als der vorbestimmte Referenzwert $DMS_R$, oder Folgern, dass der Patient nicht auf den DNMTi antworten wird, wenn der DMS-Score niedrieger ist als der vorbestimmte Referenzwert $DMS_R$.

2. Verfahren gemäß Anspruch 1, wobei der DNA-Methyltransferase-Inhibitor ein Nukleosidanalogon ist.

3. Verfahren gemäß Anspruch 2, wobei das Nukleosidanalogon ausgewählt ist aus der Gruppe bestehend aus 5-Azacytidin (Azacytidin), 5-Aza-2'-desoxycytidin (Decitabin, 5-Aza-CdR), Zebularin, 5-Fluor-2'-desoxycytidin (5-F-CdR), 5,6-Dihydro-5-azacytidin (DHAC).

4. Verfahren gemäß Anspruch 2, wobei der DNA-Methyltransferase-Inhibitor ein Nicht-Nukleosid-Analogon ist.

5. Verfahren gemäß Anspruch 4, wobei das Nicht-Nukleosid-Analogon ausgewählt ist aus der Gruppe bestehend aus Hydralazin, Procainamid, Procain, EGCG ((-)-Epigallocatechin-3-gallat), Psammaplin A, MG98, RG108.

6. DNA-Methyltransferase-Inhibitor zur Verwendung in der Behandlung von multiplem Myelom in einem Patienten, der einer solchen bedarf, wobei der Patient mit einem Verfahren gemäß Anspruch 1 getestet wurde, wobei der vorbestimmte Referenzwert $DSM_R$ der DSM-Mittelwert ist, und wobei der Patient anhand des Verfahrens gemäß Anspruch 1 als Responder klassifiziert ist.

7. DNA-Methyltransferase-Inhibitor zur Verwendung gemäß Anspruch 6, wobei der DNA-Methyltransferase-Inhibitor ein Nukleosid-Analogon ist.

8. DNA-Methyltransferase-Inhibitor zur Verwendung gemäß Anspruch 7, wobei das Nukleosidanalogon ausgewählt ist aus der Gruppe bestehend aus 5-Azacytidin (Azacytidin), 5-Aza-2'-desoxycytidin (Decitabin, 5-Aza-CdR), Zebularin, 5-Fluor-2'-desoxycytidin (5-F-CdR), 5,6-Dihydro-5-azacytidin (DHAC).

9. DNA-Methyltransferase-Inhibitor zur Verwendung gemäß Anspruch 6, wobei der DNA-Methyltransferase-Inhibitor ein Nicht-Nukleosid-Analogon ist.

10. DNA-Methyltransferase-Inhibitor zur Verwendung gemäß Anspruch 9, wobei die Nicht-Nukleosid-Analoga ausgewählt sind aus der Gruppe bestehend aus Hydralazin, Procainamid, Procain, EGCG ((-)-Epigallocatechin-3-gallat), Psammaplin A, MG98, RG108.

**Revendications**

1. Un procédé pour tester si un patient souffrant d'un myélome multiple répondra ou non à un inhibiteur de méthyltransférases d'ADN (DNMTi) consistant à :

i. déterminer le niveau d'expression (ELi) des 47 gènes $G_1$ à $G_{47}$ à partir du tableau A dans un échantillon biologique obtenu dudit patient

ii. comparer le niveau d'expression (ELi) déterminé à l'étape i) avec un niveau de référence prédéterminé (ELRi)

iii. calculer le score DMS à l'aide de la formule suivante

$$DMS = \sum_{i=1}^{n} \beta i \times Ci$$

dans laquelle $\beta i$ représente la valeur de référence du coefficient de régression $\beta$ pour le gène $G_i$ et $Ci = 1$ si l'expression du gène $G_i$ (ELi) est supérieure au niveau de référence prédéterminé (ELRi) ou $Ci = -1$ si l'expression du gène (ELi) est inférieure ou égale au niveau de référence prédéterminé (ELRi)

iv. comparer le score DMS déterminé à l'étape iii) avec une valeur de référence prédéterminée DMS$_R$

v. et conclure que le patient répondra au DNMTi lorsque le score DMS est supérieur à la valeur de référence prédéterminée DMS$_R$ ou conclure que le patient ne répondra pas au DNMTi lorsque le score DMS est inférieur à la valeur de référence prédéterminée DMSR.

2. Procédé selon la revendication 1, dans lequel ledit inhibiteur de méthyltransférases d'ADN est un analogue nucléosidique.

3. Procédé selon la revendication 2, dans lequel l'analogue nucléosidique est choisi dans le groupe constitué par la 5-Azacytidine (azacytidine), la 5-Aza-2'-désoxycytidine (décitabine, 5-Aza-CdR), la zébularine, la 5-Fluoro-2'-déoxycytidine (5-F-CdR) et la 5,6-dihydro-5-azacytidine (DHAC).

4. Procédé selon la revendication 2, dans lequel ledit inhibiteur de méthyltransférases d'ADN est un analogue non nucléosidique.

5. Procédé selon la revendication 4, dans lequel l'analogue non nucléosidique est choisi dans le groupe constitué par l'Hydralazine, la Procaïnamide, la Procaïne, l'EGCG ((-)-épigallocatéchine-3-gallate), la Psammapline A, MG98 et RG108.

6. Un inhibiteur de méthyltransférases d'ADN pour son utilisation dans le traitement du myélome multiple chez un patient qui en a besoin, le patient ayant été testé suivant le procédé selon la revendication 1, la valeur de référence prédéterminée DMS$_R$ étant la valeur médiane de DMS, et ledit patient étant classé comme répondant par le procédé selon la revendication 1.

7. Inhibiteur de méthyltransférases d'ADN pour son utilisation selon la revendication 6, dans lequel ledit inhibiteur de méthyltransférases d'ADN est un analogue nucléosidique.

8. Inhibiteur de méthyltransférases d'ADN pour son utilisation selon la revendication 7, dans lequel les analogues nucléosidiques sont choisis dans le groupe constitué par la 5-Azacytidine (azacytidine), la 5-Aza-2'-désoxycytidine (décitabine, 5-Aza-CdR), la zébularine, la 5-Fluoro-2'-désoxycytidine (5-F- CdR) et la 5,6-dihydro-5-azacytidine (DHAC).

9. Inhibiteur de méthyltransférases d'ADN pour son utilisation selon la revendication 6, dans lequel ledit inhibiteur de méthyltransférases d'ADN est un analogue non nucléosidique.

10. Inhibiteur de méthyltransférases d'ADN pour son utilisation selon la revendication 9, dans lequel les analogues non nucléosidiques sont choisis dans le groupe constitué par l'Hydralazine, la Procaïnamide, la Procaïne, l'EGCG ((-)-épigallocatéchine-3-gallate), la Psammapline A, MG98 et RG108.

Figure 1A

39

Figure 1B

**HM cohort (N = 206)**

DM Score ≤ -15.3
N = 135 (65.5%)

$P = 2.1E\text{-}19$

DM Score > -15.3
N = 71 (34.5%)

**TT2 cohort (N = 345)**

DM Score ≤ -15.3
N = 256 (74.2%)

$P = 1E\text{-}4$

DM Score > -15.3
N = 89 (25.8%)

**Figure 2**

| HMCLs | | IC50 μM | DM Score | |
|---|---|---|---|---|
| ▽ | LP1 | 60.81 | -9.64 | |
| ○ | XG13 | 21.96 | -8.75 | |
| △ | XG6 | 7.94 | -15.21 | Low DM Score |
| □ | XG20 | 6.84 | -5.29 | |
| ◇ | SKMM2 | 2.92 | -8.46 | |
| ● | XG16 | 2.22 | 5.38 | |
| ◆ | JJN3 | 1.34 | 4.68 | |
| ▼ | RPMI | .68 | 4.38 | High DM Score |
| ■ | XG12 | .15 | 7.55 | |
| ▲ | XG19 | .15 | 10.61 | |

$P = .01$

Figure 3

Figure 4

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HOHEISEL.** *Nature Reviews, Genetics,* 2006, vol. 7, 200-210
- **HAHN WC ; WEINBERG RA.** Rules for making human tumor cells. *N Engl J Med.,* 2002, vol. 347 (20), 1593-1603
- **VOGELSTEIN B ; KINZLER KW.** Cancer genes and the pathways they control. *Nat Med.,* 2004, vol. 10 (8), 789-799
- **BAYLIN SB.** DNA methylation and gene silencing in cancer. *Nat Clin Pract Oncol.,* 2005, vol. 2 (1), 4-11
- **KONDO Y.** Epigenetic cross-talk between DNA methylation and histone modifications in human cancers. *Yonsei Med J.,* 2009, vol. 50 (4), 455-463
- **ISSA JP.** DNA methylation as a therapeutic target in cancer. *Clin Cancer Res.,* 2007, vol. 13 (6), 1634-1637
- **ISSA JP ; GARCIA-MANERO G ; GILES FJ et al.** Phase 1 study of low-dose prolonged exposure schedules of the hypomethylating agent 5-aza-2'-deoxycytidine (decitabine) in hematopoietic malignancies. *Blood,* 2004, vol. 103 (5), 1635-1640
- **OKI Y ; JELINEK J ; SHEN L ; KANTARJIAN HM ; ISSA JP.** Induction of hypomethylation and molecular response after decitabine therapy in patients with chronic myelomonocytic leukemia. *Blood,* 2008, vol. 111 (4), 2382-2384
- **SMITH EM ; BOYD K ; DAVIES FE.** The potential role of epigenetic therapy in multiple myeloma. *Br J Haematol.,* 2009
- **BERGSAGEL PL ; KUEHL WM.** Molecular pathogenesis and a consequent classification of multiple myeloma. *J Clin Oncol.,* 2005, vol. 23 (26), 6333-6338
- **HIDESHIMA T ; BERGSAGEL PL ; KUEHL WM ; ANDERSON KC.** Advances in biology of multiple myeloma: clinical applications. *Blood,* 2004, vol. 104 (3), 607-618
- **CHEN G ; WANG Y ; HUANG H et al.** Combination of DNA methylation inhibitor 5-azacytidine and arsenic trioxide has synergistic activity in myeloma. *Eur J Haematol.,* 2009, vol. 82 (3), 176-183
- **DE CARVALHO F ; COLLEONI GW ; ALMEIDA MS ; CARVALHO AL ; VETTORE AL.** TGFbetaR2 aberrant methylation is a potential prognostic marker and therapeutic target in multiple myeloma. *Int J Cancer.,* 2009, vol. 125 (8), 1985-1991
- **GALM O ; YOSHIKAWA H ; ESTELLER M ; OSIEKA R ; HERMAN JG.** SOCS-1, a negative regulator of cytokine signaling, is frequently silenced by methylation in multiple myeloma. *Blood,* 2003, vol. 101 (7), 2784-2788
- **HATZIMICHAEL E ; DRANITSARIS G ; DASOULA A et al.** Von Hippel-Lindau methylation status in patients with multiple myeloma: a potential predictive factor for the development of bone disease. *Clin Lymphoma Myeloma,* 2009, vol. 9 (3), 239-242
- **HODGE DR ; PENG B ; CHERRY JC et al.** Interleukin 6 supports the maintenance of p53 tumor suppressor gene promoter methylation. *Cancer Res.,* 2005, vol. 65 (11), 4673-4682
- **NG MH ; CHUNG YF ; LO KW ; WICKHAM NW ; LEE JC ; HUANG DP.** Frequent hypermethylation of p16 and p15 genes in multiple myeloma. *Blood,* 1997, vol. 89 (7), 2500-2506
- **SEIDL S ; ACKERMANN J ; KAUFMANN H et al.** DNA-methylation analysis identifies the E-cadherin gene as a potential marker of disease progression in patients with monoclonal gammopathies. *Cancer,* 2004, vol. 100 (12), 2598-2606
- **TSHUIKINA M ; JERNBERG-WIKLUND H ; NILSSON K ; OBERG F.** Epigenetic silencing of the interferon regulatory factor ICSBP/IRF8 in human multiple myeloma. *Exp Hematol.,* 2008, vol. 36 (12), 1673-1681
- **HELLER G ; SCHMIDT WM ; ZIEGLER B et al.** Genome-wide transcriptional response to 5-aza-2'-deoxycytidine and trichostatin a in multiple myeloma cells. *Cancer Res.,* 2008, vol. 68 (1), 44-54
- **WILOP S ; VAN GEMMEREN TB ; LENTJES MH et al.** Methylation-associated dysregulation of the suppressor of cytokine signaling-3 gene in multiple myeloma. *Epigenetics,* 2011, vol. 6 (8), 1047-1052
- **WALKER BA ; WARDELL CP ; CHIECCHIO L et al.** Aberrant global methylation patterns affect the molecular pathogenesis and prognosis of multiple myeloma. *Blood,* 2010
- **MOREAUX J ; KLEIN B ; BATAILLE R et al.** A high-risk signature for patients with multiple myeloma established from the molecular classification of human myeloma cell lines. *Haematologica,* 2011, vol. 96 (4), 574-582

- **ZHANG XG ; GAILLARD JP ; ROBILLARD N et al.** Reproducible obtaining of human myeloma cell lines as a model for tumor stem cell study in human multiple myeloma. *Blood,* 1994, vol. 83 (12), 3654-3663
- **REBOUISSOU C ; WIJDENES J ; AUTISSIER P et al.** A gp130 interleukin-6 transducer-dependent SCID model of human multiple myeloma. *Blood,* 1998, vol. 91 (12), 4727-4737
- **TARTE K ; ZHANG XG ; LEGOUFFE E et al.** Induced expression of B7-1 on myeloma cells following retroviral gene transfer results in tumor-specific recognition by cytotoxic T cells. *J Immunol.,* 1999, vol. 163 (1), 514-524
- **GU ZJ ; VOS JD ; REBOUISSOU C et al.** Agonist anti-gp130 transducer monoclonal antibodies are human myeloma cell survival and growth factors. *Leukemia,* 2000, vol. 14 (1), 188-197
- **GOLDSCHMIDT H ; SONNEVELD P ; CREMER FW et al.** Joint HOVON-50/GMMG-HD3 randomized trial on the effect of thalidomide as part of a high-dose therapy regimen and as maintenance treatment for newly diagnosed myeloma patients. *Ann Hematol.,* 2003, vol. 82 (10), 654-659
- **CREMER FW ; BILA J ; BUCK I et al.** Delineation of distinct subgroups of multiple myeloma and a model for clonal evolution based on interphase cytogenetics. *Genes Chromosomes Cancer.,* 2005, vol. 44 (2), 194-203
- **BARLOGIE B ; TRICOT G ; RASMUSSEN E et al.** Total therapy 2 without thalidomide in comparison with total therapy 1: role of intensified induction and posttransplantation consolidation therapies. *Blood,* 2006, vol. 107 (7), 2633-2638
- **SPRYNSKI AC ; HOSE D ; CAILLOT L et al.** The role of IGF-1 as a major growth factor for myeloma cell lines and the prognostic relevance of the expression of its receptor. *Blood,* 2009, vol. 113 (19), 4614-4626
- **XIONG W ; WU X ; STARNES S et al.** An analysis of the clinical and biologic significance of TP53 loss and the identification of potential novel transcriptional targets of TP53 in multiple myeloma. *Blood,* 2008, vol. 112 (10), 4235-4246
- **MOREAUX J ; LEGOUFFE E ; JOURDAN E et al.** BAFF and APRIL protect myeloma cells from apoptosis induced by interleukin 6 deprivation and dexamethasone. *Blood,* 2004, vol. 103 (8), 3148-3157
- **HOSE D ; REME T ; MEISSNER T et al.** Inhibition of aurora kinases for tailored risk-adapted treatment of multiple myeloma. *Blood,* 2009, vol. 113 (18), 4331-4340
- **MOREAUX J ; CREMER FW ; REME T et al.** The level of TACI gene expression in myeloma cells is associated with a signature of microenvironment dependence versus a plasmablastic signature. *Blood,* 2005, vol. 106 (3), 1021-1030
- **REME T ; HOSE D ; DE VOS J et al.** A new method for class prediction based on signed-rank algorithms applied to Affymetrix microarray experiments. *BMC Bioinformatics,* 2008, vol. 9, 16
- **TANGUY LE CARROUR SA ; SYLVIE TONDEUR ; LUDOVIC LHERMITTE ; NED LAMB ; THIERRY REME ; VERONIQUE PANTESCO ; SAMIR HAMAMAH ; BERNARD KLEIN ; JOHN DE VOS.** Amazonia!: An Online Resource to Google and Visualize Public Human whole Genome Expression Data. *The Open Bioinformatics Journal,* 2010, vol. 4, 5-10
- **CUI X ; CHURCHILL GA.** Statistical tests for differential expression in cDNA microarray experiments. *Genome Biol.,* 2003, vol. 4 (4), 210
- **KASSAMBARA A ; HOSE D ; MOREAUX J et al.** Genes with a spike expression are clustered in chromosome (sub)bands and spike (sub)bands have a powerful prognostic value in patients with multiple myeloma. *Haematologica,* 2011
- **ZHAN F ; HUANG Y ; COLLA S et al.** The molecular classification of multiple myeloma. *Blood,* 2006, vol. 108 (6), 2020-2028
- **CHU WK ; HICKSON ID.** RecQ helicases: multifunctional genome caretakers. *Nat Rev Cancer,* 2009, vol. 9 (9), 644-654
- **HARRIGAN JA ; BOHR VA.** Human diseases deficient in RecQ helicases. *Biochimie,* 2003, vol. 85 (11), 1185-1193
- **HICKSON ID.** RecQ helicases: caretakers of the genome. *Nat Rev Cancer,* 2003, vol. 3 (3), 169-178
- **ARAI A ; CHANO T ; FUTAMI K et al.** RECQL1 and WRN proteins are potential therapeutic targets in head and neck squamous cell carcinoma. *Cancer Res.,* 2011, vol. 71 (13), 4598-4607
- **FUTAMI K ; KUMAGAI E ; MAKINO H et al.** Induction of mitotic cell death in cancer cells by small interference RNA suppressing the expression of RecQL1 helicase. *Cancer Sci.,* 2008, vol. 99 (1), 71-80
- **FUTAMI K ; KUMAGAI E ; MAKINO H et al.** Anticancer activity of RecQL1 helicase siRNA in mouse xenograft models. *Cancer Sci.,* 2008, vol. 99 (6), 1227-1236
- **MENDOZA-MALDONADO R ; FAORO V ; BAJPAI S et al.** The human RECQ1 helicase is highly expressed in glioblastoma and plays an important role in tumor cell proliferation. *Mol Cancer.,* 2011, vol. 10, 83
- **MIKI H ; OKADA Y ; HIROKAWA N.** Analysis of the kinesin superfamily: insights into structure and function. *Trends Cell Biol.,* 2005, vol. 15 (9), 467-476
- **HIROKAWA N.** Kinesin and dynein superfamily proteins and the mechanism of organelle transport. *Science,* 1998, vol. 279 (5350), 519-526

- YU Y ; FENG YM. The role of kinesin family proteins in tumorigenesis and progression: potential biomarkers and molecular targets for cancer therapy. *Cancer,* 2010, vol. 116 (22), 5150-5160
- ZHU C ; ZHAO J ; BIBIKOVA M et al. Functional analysis of human microtubule-based motor proteins, the kinesins and dyneins, in mitosis/cytokinesis using RNA interference. *Mol Biol Cell,* 2005, vol. 16 (7), 3187-3199
- AVET-LOISEAU H ; LI C ; MAGRANGEAS F et al. Prognostic significance of copy-number alterations in multiple myeloma. *J Clin Oncol.,* 2009, vol. 27 (27), 4585-4590
- AGNELLI L ; FORCATO M ; FERRARI F et al. The reconstruction of transcriptional networks reveals critical genes with implications for clinical outcome of multiple myeloma. *Clin Cancer Res.,* 2011, vol. 17 (23), 7402-7412
- DE VOS, J. ; THYKJAER, T. ; TARTE, K. ; ENSSLEN, M. ; RAYNAUD, P. ; REQUIRAND, G. ; PELLET, F. ; PANTESCO, V. ; REME, T. ; JOURDAN, M. Comparison of gene expression profiling between malignant and normal plasma cells with oligonucleotide arrays. *Oncogene,* 2002, vol. 21, 6848-6857
- FLOTHO, C. ; CLAUS, R. ; BATZ, C. ; SCHNEIDER, M. ; SANDROCK, I. ; IHDE, S. ; PLASS, C. ; NIEMEYER, C.M. ; LUBBERT, M. The DNA methyltransferase inhibitors azacitidine, decitabine and zebularine exert differential effects on cancer gene expression in acute myeloid leukemia cells. *Leukemia,* 2009, vol. 23, 1019-1028
- GHOSHAL, K. ; DATTA, J. ; MAJUMDER, S. ; BAI, S. ; KUTAY, H. ; MOTIWALA, T. ; JACOB, S.T. 5-Aza-deoxycytidine induces selective degradation of DNA methyltransferase 1 by a proteasomal pathway that requires the KEN box, bromo-adjacent homology domain, and nuclear localization signal. *Mol Cell Biol,* 2005, vol. 25, 4727-4741
- HEUCK, C.J. ; MEHTA, J. ; BHAGAT, T. ; GUNDABOLU, K. ; YU, Y. ; KHAN, S. ; CHRYSOFAKIS, G. ; SCHINKE, C. ; TARIMAN, J. ; VICKREY, E. Myeloma is characterized by stage-specific alterations in DNA methylation that occur early during myelomagenesis. *J Immunol,* 2013, vol. 190, 2966-2975
- HOLLENBACH, P.W. ; NGUYEN, A.N. ; BRADY, H. ; WILLIAMS, M. ; NING, Y. ; RICHARD, N. ; KRUSHEL, L. ; AUKERMAN, S.L. ; HEISE, C. ; MACBETH, K.J. A comparison of azacitidine and decitabine activities in acute myeloid leukemia cell lines. *PLoS One,* 2010, vol. 5, e9001
- JOURDAN, M. ; FERLIN, M. ; LEGOUFFE, E. ; HORVATHOVA, M. ; LIAUTARD, J. ; ROSSI, J.F. ; WIJDENES, J. ; BROCHIER, J. ; KLEIN, B. The myeloma cell antigen syndecan-1 is lost by apoptotic myeloma cells. *Br J Haematol,* 1998, vol. 100, 637-646
- KAISER, M.F. ; JOHNSON, D.C. ; WU, P. ; WALKER, B.A. ; BRIOLI, A. ; MIRABELLA, F. ; WARDELL, C.P. ; MELCHOR, L. ; DAVIES, F.E. ; MORGAN, G.J. Global methylation analysis identifies prognostically important epigenetically inactivated tumour suppressor genes in multiple myeloma. *Blood,* 2013
- KIZILTEPE, T. ; HIDESHIMA, T. ; CATLEY, L. ; RAJE, N. ; YASUI, H. ; SHIRAISHI, N. ; OKAWA, Y. ; IKEDA, H. ; VALLET, S. ; POZZI, S. 5-Azacytidine, a DNA methyltransferase inhibitor, induces ATR-mediated DNA double-strand break responses, apoptosis, and synergistic cytotoxicity with doxorubicin and bortezomib against multiple myeloma cells. *Mol Cancer Ther,* 2007, vol. 6, 1718-1727
- MAES, K. ; MENU, E. ; VAN VALCKENBORGH, E. ; VAN RIET, I. ; VANDERKERKEN, K. ; DE BRUYNE, E. Epigenetic Modulating Agents as a New Therapeutic Approach in Multiple Myeloma. *Cancers,* 2013, vol. 5, 430-461
- MAHTOUK, K. ; JOURDAN, M. ; DE VOS, J. ; HERTOGH, C. ; FIOL, G. ; JOURDAN, E. ; ROSSI, J.F. ; KLEIN, B. An inhibitor of the EGF receptor family blocks myeloma cell growth factor activity of HB-EGF and potentiates dexamethasone or anti-IL-6 antibody-induced apoptosis. *Blood,* 2004, vol. 103, 1829-1837
- MOREAUX, J. ; REME, T. ; LEONARD, W. ; VEYRUNE, J.L. ; REQUIRAND, G. ; GOLDSCHMIDT, H. ; HOSE, D. ; KLEIN, B. Development of gene expression-based score to predict sensitivity of multiple myeloma cells to DNA methylation inhibitors. *Mol Cancer Ther,* 2012, vol. 11, 2685-2692
- PALII, S.S. ; VAN EMBURGH, B.O. ; SANKPAL, U.T. ; BROWN, K.D. ; ROBERTSON, K.D. DNA methylation inhibitor 5-Aza-2'-deoxycytidine induces reversible genome-wide DNA damage that is distinctly influenced by DNA methyltransferases 1 and 3B. *Mol Cell Biol,* 2008, vol. 28, 752-771
- RODRIGUEZ-PAREDES, M. ; ESTELLER, M. Cancer epigenetics reaches mainstream oncology. *Nat Med,* 2011, vol. 17, 330-339
- STRESEMANN, C. ; BRUECKNER, B. ; MUSCH, T. ; STOPPER, H. ; LYKO, F. Functional diversity of DNA methyltransferase inhibitors in human cancer cell lines. *Cancer Res,* 2006, vol. 66, 2794-2800
- TANGUY LE CARROUR, S.A. ; SYLVIE TONDEUR ; LUDOVIC LHERMITTE ; NED LAMB ; THIERRY REME ; VERONIQUE PANTESCO ; SAMIR HAMAMAH ; BERNARD KLEIN ; JOHN DE VOS. Amazonia!: An Online Resource to Google and Visualize Public Human whole Genome Expression Data. *The Open Bioinformatics Journal,* 2010, vol. 4, 5-10

- **TOOR, A.A. ; PAYNE, K.K. ; CHUNG, H.M. ; SABO, R.T. ; HAZLETT, A.F. ; KMIECIAK, M. ; SANFORD, K. ; WILLIAMS, D.C. ; CLARK, W.B. ; ROBERTS, C.H.** Epigenetic induction of adaptive immune response in multiple myeloma: sequential azacitidine and lenalidomide generate cancer testis antigen-specific cellular immunity. *Br J Haematol,* 2012, vol. 158, 700-711
- **WALKER, B.A. ; WARDELL, C.P. ; CHIECCHIO, L. ; SMITH, E.M. ; BOYD, K.D. ; NERI, A. ; DAVIES, F.E. ; ROSS, F.M. ; MORGAN, G.J.** Aberrant global methylation patterns affect the molecular pathogenesis and prognosis of multiple myeloma. *Blood,* 2010

- **ZHANG, X.G. ; GAILLARD, J.P. ; ROBILLARD, N. ; LU, Z.Y. ; GU, Z.J. ; JOURDAN, M. ; BOIRON, J.M. ; BATAILLE, R. ; KLEIN, B.** Reproducible obtaining of human myeloma cell lines as a model for tumor stem cell study in human multiple myeloma. *Blood,* 1994, vol. 83, 3654-3663
- Epigenetic changes of myeloma cells within the bone marrow microenvironment. **VANDERKERKEN, K. et al.** Haematologica, Abstract Book 13th International Myeloma Workshop. 2011, vol. 96
- **SMITH, E.M. et al.** The potential role of epigenetic therapy in multiple myeloma. *Br J Haematol,* 01 March 2010, vol. 148 (5), 702-713